# EUROPEAN PATENT APPLICATION

(11) **EP 4 588 917 A1**
(43) Date of publication of application: **23.07.2025**
(21) Application number: 23864819.0
(22) Date of filing: 15.09.2023
(51) Int. Cl.: C07D 471/04, C07D 475/00, A61K 31/519, A61P 35/02

(54) **PYRIDONOPYRIMIDINE DERIVATIVE AS RSK INHIBITOR AND USE THEREOF**

(30) Priority: 16.09.2022 CN 202211134803
(71) Applicant: East China Normal University, Shanghai 200241 (CN); East China University of Science and Technology, Shanghai 200237 (CN); Zhengzhou University, Zhengzhou, Henan 450001 (CN); Jinfeng Laboratory, Chongqing 401329 (CN)
(72) Inventor: LI, Honglin, Shanghai 200241 (CN); LI, Shiliang, Shanghai 200241 (CN); QIAN, Xuhong, Shanghai 200241 (CN); LIU, Kangdong, Zhengzhou, Henan 450001 (CN); WANG, Zhe, Chongqing 401329 (CN); ZHAO, Zhenjiang, Shanghai 200237 (CN); XU, Yufang, Shanghai 200237 (CN); HE, Huan, Shanghai 200241 (CN)
(74) Representative: Mewburn Ellis LLP
(86) International application number: PCT/CN2023/119237
(87) International publication number: WO 2024/056091

(57) **Abstract**

The present invention relates to a pyridonopyrimidine derivative as an RSK protein kinase inhibitor and use thereof. Specifically, the present invention relates to a compound represented by formula I, a pharmaceutical composition comprising the compound represented by formula I, and use of the compound in preparing a medicament for treating an RSK-related disease or inhibiting RSK.

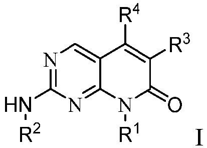

## Description

### Technical field

The present invention relates to the field of medicinal chemistry. In particular, the present invention relates to a novel pyridonopyrimidine derivative, synthesis methods, and uses thereof as a RSK inhibitor in the preparation of a drug for tumor-related diseases.

### Background

Cardiovascular diseases, cancers, respiratory diseases, and diabetes are main components of non-communicable diseases (NCDs). Currently, the main causes of human death are non-communicable diseases, and among them, the cardiovascular disease causes the largest number of human deaths, while the cancer is the second leading cause of death related to human survival. There are mainly three traditional means for treating a cancer: surgery, radiotherapy, and chemotherapy. Recently, with the development of science and technology, a targeted therapy and immunotherapy have gradually become effective means for treating a cancer. The targeted therapy mainly involves a therapy by small molecule drugs or monoclonal antibodies, which control the growth and spread of tumor cells in the body by interfering with specific proteins, thereby treating a cancer. As researchers learn more about cancer-related proteins, it is promising to design small molecule drugs targeting such proteins for treating a cancer. The Ras-MAPK signaling pathway is involved in regulating various cancer types. As the most downstream effector factor in the Ras-MAPK signaling pathway, the abnormal expression and activity of RSK are related to the occurrence and development of various diseases. The Ras-MAPK signaling pathway is activated under the stimulation by growth factors, mitogenic hormones, and neurotransmitters. The activation of cell surface receptors will enhance the autophosphorylation of Tyr kinases and generate docking sites for growth factor receptor-bound protein 2 (GRB2), which connects the receptor to the serum-free guanine nucleotide exchange factor (SOS). SOS catalyzes the binding of GTP to Ras. GTP binds to Ras and activates its effector Raf kinase. Raf is phosphorylated and activates MAPK and extracellular signal-regulated kinase (MEK1/2). Ribosomal S6 kinase (RSK) is directly phosphorylated and activated by ERK1/2 and 3-phosphoinositide-dependent kinase-1 (PDK1). The activated RSK remains membrane-associated, free in the cytoplasm, or translocated into the nucleus, thereby mediating cell differentiation, proliferation, survival, and transformation of oncogenes. The Ras signaling pathway promotes cell proliferation and protects cells from apoptosis, thereby playing a significant role in the occurrence, development, and maintenance of biological behavior of human tumors.

P90 ribosomal S6 kinase (RSK) is a member of the serine/threonine kinase family which is widely expressed in tissues, and, as an important regulatory factor downstream of the Ras signaling pathway, plays an important role in the occurrence and development of tumors. In mammals, there are four subtypes: RSK1, RSK2, RSK3, and RSK4. All of four subtypes have two functionally different kinase domains: the N-terminal kinase domain (NTKD) and the C-terminal kinase domain (CTKD), as well as a linker region. The C-terminal tail contains a specific extracellular signal-regulated kinase (ERK) binding site. After binding with ERK, this site further places RSK under the regulation of ERK.

At present, there are no drugs targeting RSK in clinical use. The RSK small molecule inhibitors under development mainly include two categories. One is RSK2 selective inhibitors, including SL0101, CMK, etc.; and the other is pan-inhibitors of RSK, including BID-1870, FMK, LJH308, LJH685, etc. None of these small molecule inhibitors have entered clinical research.

Therefore, researching and developing drugs targeting RSK has significant clinical significance and application prospects.

### Summary of the invention

The purpose of the present invention is to provide pyridonopyrimidine derivatives as RSK inhibitors.

Another purpose of the present invention is to provide a pharmaceutical composition comprising the compound as said above.

Yet another purpose of the present invention is to provide the use of the compound as said above in the preparation of a medicament for treating RSK-related diseases or inhibiting RSK.

In the first aspect, a compound as shown in Formula I, or an optical isomer, or pharmaceutically acceptable salt thereof is provided in the present invention: 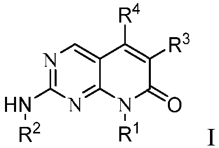
R¹ is selected from the group consisting of: a hydrogen, optionally substituted C₁-C₁₀ alkyl, optionally substituted C₃-C₈ cycloalkyl, optionally substituted C₂-C₆ alkenyl, optionally substituted C₃-C₆ cycloalkenyl, optionally substituted C₃-C₈ lactone group, optionally substituted C₁-C₁₀ amide group, optionally substituted C₅-C₁₀ aryl, optionally substituted C₃-C₈ heterocyclyl, optionally substituted C₅-C₁₀ heteroaryl;
R² is selected from the group consisting of: a hydrogen, substituted C₁-C₁₀ alkyl, C₃-C₈ cycloalkyl, optionally substituted C₅-C₁₀ aryl, optionally substituted C₃-C₈ heterocyclyl, optionally substituted C₅-C₁₀ aryl or heteroaryl fused to a five- or six- membered heterocycle;
R³ is selected from the group consisting of: a hydrogen, substituted C₁-C₁₀ alkyl, C₃-C₈ cycloalkyl, substituted C₁-C₁₀ alkyl formyl, optionally substituted C₅-C₁₀ aryl formyl, halogen, cyano, optionally substituted C₅-C₁₀ aryl, optionally substituted C₃-C₈ heterocyclyl;
R⁴ is selected from the group consisting of: a hydrogen, optionally substituted C₁-C₆ alkyl (for example, trifluoromethyl), optionally substituted C₃-C₈ cycloalkyl, optionally substituted C₁-C₁₀ alkyl formyl, optionally substituted C₅-C₁₀ aryl formyl, optionally substituted C₅-C₁₀ aryl, halogen (for example, fluorine) and optionally substituted C₃-C₈ heterocyclyl.

In a preferred embodiment, the aryl or heteroaryl fused to a five- or six- membered heterocycle is a phenyl fused to a five- or six- membered heterocycle, including but not limited to:

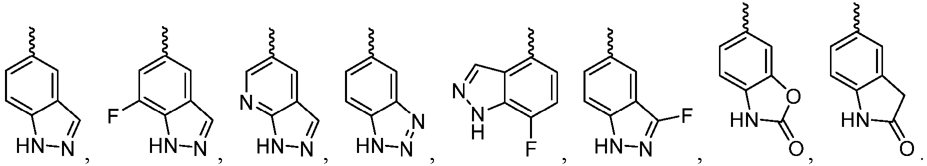

In a specific embodiment, R¹ is selected from the group consisting of: a hydrogen, optionally substituted C₁-C₁₀ alkyl, optionally substituted C₃-C₈ cycloalkyl, optionally substituted C₅-C₁₀ aryl, and optionally substituted C₃-C₈ heterocyclyl;
R² is selected from the group consisting of: an optionally substituted C₅-C₁₀ aryl, optionally substituted C₃-C₈ heterocyclyl, and optionally substituted C₅-C₁₀ aryl or heteroaryl fused to a five- or six- membered heterocycle;
R³ is selected from the group consisting of: a hydrogen, optionally substituted C₁-C₁₀ alkyl, and optionally substituted C₃-C₈ cycloalkyl;
R⁴ is selected from the group consisting of: a hydrogen, optionally substituted C₁-C₆ alkyl (for example, trifluoromethyl), optionally substituted C₃-C₈ cycloalkyl, halogen (for example, fluorine), and optionally substituted C₃-C₈ heterocyclyl.

In a specific embodiment, the compound is a compound as shown in Formula II, 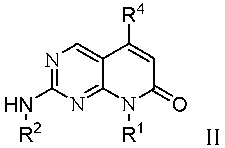
Wherein R¹ is selected from the group consisting of: a hydrogen, optionally substituted C₁-C₁₀ alkyl, optionally substituted C₃-C₈ cycloalkyl;
R² is selected from the group consisting of: an optionally substituted C₅-C₁₀ aryl, optionally substituted C₃-C₈ heterocyclyl, optionally substituted C₅-C₁₀ aryl or heteroaryl fused to a five- or six- membered heterocycle;
R⁴ is selected from the group consisting of: a hydrogen, optionally substituted C₁-C₆ alkyl (for example, trifluoromethyl), optionally substituted C₃-C₈ cycloalkyl.

In a specific embodiment, R¹ is an optionally substituted C₃-C₈ cycloalkyl; preferably, a C₃-C₈ cycloalkyl substituted with one or more halogens; and more preferably, a C₃-C₈ cycloalkyl substituted with one or more F;
R² is selected from the following group: an optionally substituted C₅-C₁₀ aryl (preferably a phenyl), an optionally substituted C5-C10 aryl or heteroaryl fused to five- or six- membered heterocycle;
the C₅-C₁₀ aryl or heteroaryl fused to five- or six- membered heterocycle is: 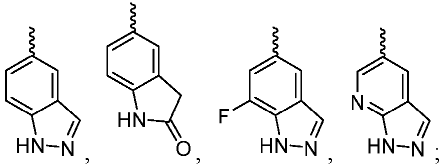
R⁴ is selected from the group consisting of: a hydrogen, optionally substituted C₁-C₆ alkyl.

In a specific embodiment, the compound is a compound selected from the following group, or an optical isomer, or pharmaceutically acceptable salt thereof: 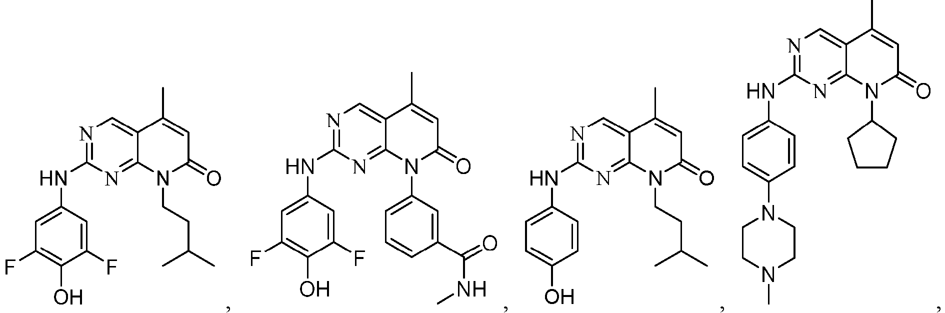 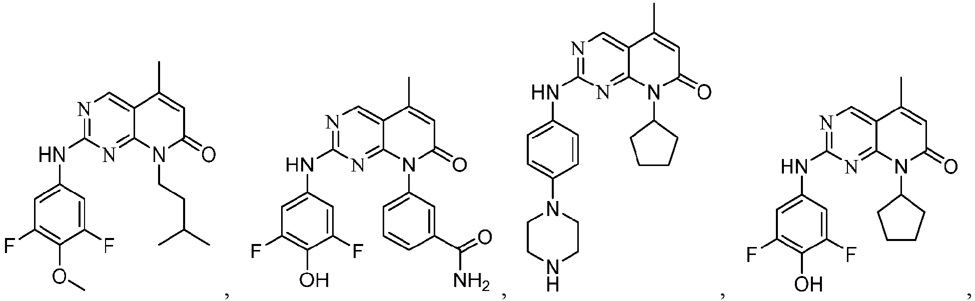 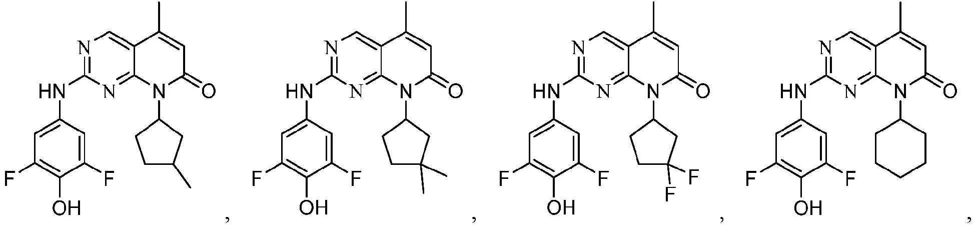 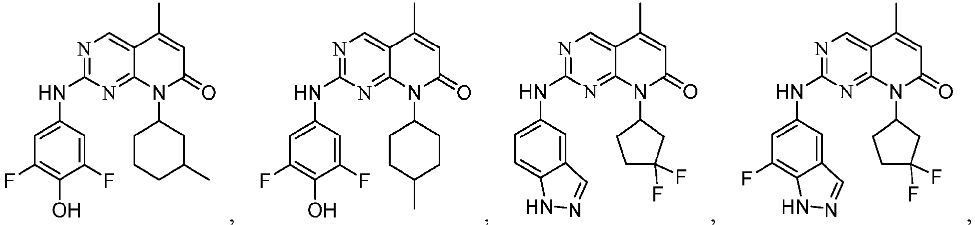 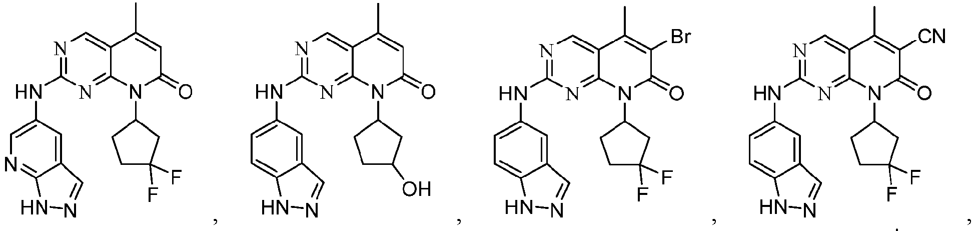 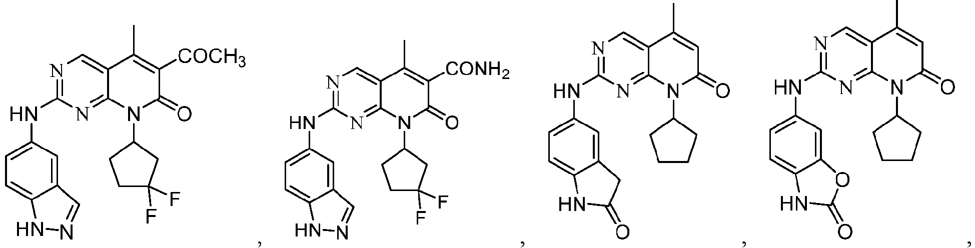 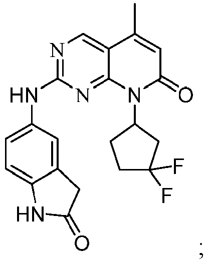 and preferably, following compounds:

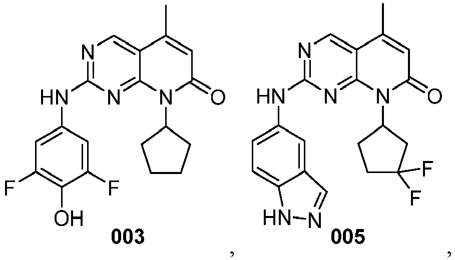

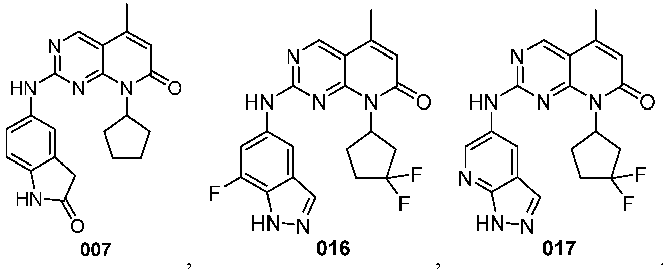

In a second aspect, a pharmaceutical composition comprising the compound as described in the first aspect, or an optical isomer, or pharmaceutically acceptable salt thereof, and a pharmaceutically acceptable carrier or excipient is provided the present invention.

In a preferred embodiment, the pharmaceutical composition is in a dosage form suitable for the oral administration, including but not limited to a tablet, solution, suspension, capsule, granules powder.

In a third aspect, the use of the compound as described in the first aspect in the preparation of a medicament for treating or preventing a disease mediated by RSK protein kinase, or inhibiting RSK protein kinase, or a medicament for inhibiting RSK of one of RSK1, RSK2, RSK3 and RSK4 is provided the present invention.

In a specific embodiment, the disease mediated by the RSK protein kinase is a cancer.

In a specific embodiment, the cancer is selected from the group consisting of esophageal cancer, renal cell carcinoma, pancreatic cancer, colon cancer, breast cancer, lung cancer, prostate cancer, ovarian cancer, endometrial cancer, head and neck squamous cell carcinoma, acute myeloid leukemia, and solid tumors, or breast cancer regulated by RSK1 and RSK4, ovarian cancer regulated by RSK3 and RSK4, prostate cancer regulated by RSK1 and RSK2, lung cancer regulated by RSK1, RSK2 and RSK4, head and neck squamous cell carcinoma and acute myeloid leukemia regulated by RSK2, esophageal cancer, renal cancer, endometrial cancer, colon cancer and other cancers and solid tumors regulated by RSK4.

In a fourth aspect, the compound as described in the first aspect, for use in treating or preventing a disease mediated by RSK protein kinase, or inhibiting RSK protein kinase, or inhibiting RSK of one of RSK1, RSK2, RSK3 and RSK4 is provided the present invention.

In a preferred embodiment, the disease mediated by the RSK protein kinase is a cancer.

In a preferred embodiment, the cancer is selected from the group consisting of esophageal cancer, renal cell carcinoma, pancreatic cancer, colon cancer, breast cancer, lung cancer, prostate cancer, ovarian cancer, endometrial cancer, head and neck squamous cell carcinoma, acute myeloid leukemia and solid tumors, or breast cancer regulated by RSK1 and RSK4, ovarian cancer regulated by RSK3 and RSK4, prostate cancer regulated by RSK1 and RSK2, lung cancer regulated by RSK1, RSK2 and RSK4, head and neck squamous cell carcinoma and acute myeloid leukemia regulated by RSK2, esophageal cancer, renal cancer, endometrial cancer, colon cancer and other cancers and solid tumors regulated by RSK4.

In a fifth aspect, a method for treating or preventing RSK-mediated diseases using the compound described in the first aspect or the pharmaceutical composition described in the second aspect is provided the present invention.

In a preferred embodiment, the RSK-mediated disease is cancer; preferably, the cancer is selected from the group consisting of: breast cancer regulated by RSK1 and RSK4, ovarian cancer regulated by RSK3 and RSK4, prostate cancer regulated by RSK1 and RSK2, lung cancer regulated by RSK1, RSK2 and RSK4, head and neck squamous cell carcinoma and acute myeloid leukemia regulated by RSK2, esophageal cancer, renal cancer, endometrial cancer, colon cancer and other cancers and solid tumors regulated by RSK4.

It should be understood that, within the scope of the present invention, the above technical features of the present invention and the technical features specifically described in the following (such as in the Examples) can be combined with each other to form new or preferred technical solutions. Such technical solutions will not be described one by one due to the limited contents.

### Description of drawings

Figure 1 shows the anti-invasion activity of compound 005 in esophageal squamous cell carcinoma;
Figure 2 shows the effect of compound 005 on the clone formation of esophageal squamous cell carcinoma cells;
Figure 3 shows the effect of compound 005 on the intracellular signaling pathways of esophageal squamous cell carcinoma cells.

### Modes for carrying out the invention

Through extensive and in-depth research, the inventors have discovered a group of pyridonopyrimidine derivatives with completely new structures. These derivatives are capable of inhibiting the activity of RSK kinases, with IC₅₀ values for RSK kinase inhibition reaching the nanomolar (nM) level. Based on this, the present invention has been completed.

### Definition on terms

Some definitions on the groups involved herein are as follows:
As used herein, "alkyl" refers to a saturated branched or straight-chain alkyl or cycloalkyl with a carbon chain length of 1-10 carbon atoms. Preferred alkyls include those having 1-5, 1-2, 1-6, 1-4, 3-8 carbon atoms, respectively. Examples of alkyl include, but are not limited to, methyl, ethyl, n-propyl, isopropyl, n-butyl, isobutyl, heptyl, etc. The alkyl can be substituted by one or more substituents, such as a halogen or haloalkyl. For example, the alkyl can be an alkyl substituted by 1-4 fluorine atoms, or the alkyl group can be an alkyl substituted by a fluoroalkyl.

As used herein, "alkenyl" generally refers to a monovalent hydrocarbon group having at least one double bond, usually containing 2-8, preferably 2-6 carbon atoms, and can be straight-chain or branched. Examples of alkenyl include, but are not limited to, vinyl, allyl, isopropenyl, butenyl, isobutenyl, hexenyl, etc.

As used herein, "ester group" generally refers to a carboxylic acid derivative having at least one ester group, usually containing 3-8, preferably 3-6 carbon atoms, and can be straight-chain or branched. Examples of ester group include, but are not limited to, methyl formate, ethyl formate, methyl acetate, ethyl acetate, propyl acetate, etc.

As used herein, "hydroxyl" refers to a straight-chain or branched alcohol group with a carbon chain length of 1-10 carbon atoms, usually containing 1-10, preferably 1-6 carbon atoms, and can be straight-chain or branched. Examples of ester hydroxyl include, but are not limited to, 1-hydroxybutane, 1-hydroxyisobutane, etc.

As used herein, "acylamino" refers to a group with the structural formula "-R'-NH-C(O)-R", where R' can be selected from a hydrogen or alkyl, and R can be selected from an alkyl, alkenyl, alkynyl, alkyl substituted with NR_{c}R_{d}, alkenyl substituted with NR_{c}R_{d} and alkynyl substituted with NR_{c}R_{d}, alkyl substituted with a halogen, alkenyl substituted with a cyano; wherein R_{c} and R_{d} can be selected from an alkyl or alkenyl.

As used herein, "aryl" refers to a monocyclic, bicyclic, or tricyclic aromatic groups containing 6 to 14 carbon atoms, including phenyl, naphthyl, phenanthryl, anthryl, indenyl, fluorenyl, tetrahydronaphthyl, dihydroidenyl, etc. The aryl may be optionally substituted with 1-5 (for example, 1, 2, 3, 4 or 5) substituents selected from the following group: a halogen, C₁₋₄ aldehyde group, C₁₋₆ alkyl, cyano, nitro, amino group, amide group, hydroxyl, hydroxymethyl, halogen-substituted alkyl (such as trifluoromethyl), halogen-substituted alkoxy (such as trifluoromethoxy), carboxyl, C₁₋₄ alkoxy, ethoxycarbonyl, N(CH₃) and C₁₋₄ acyl, heterocyclic groups or heteroaryl, etc.

As used herein, "heterocyclyl" includes, but not limited to, a 5- or 6- membered heterocyclic group containing 1-3 heteroatoms selected from O, S or N, including, but not limited to, furyl, thienyl, pyrrolyl, pyrrolidinyl, pyrazolyl, imidazolyl, triazolyl, oxazolyl, pyranonyl, pyridinyl, pyrimidinyl, pyrazinyl, piperidinyl, morpholinyl, etc.

As used herein, "heteroaryl" refers to a group containing 5-14 ring atoms, with 6, 10 or 14 electrons being shared in the ring system. Moreover, the ring atoms consist of carbon atoms and 1-3 heteroatoms selected from oxygen, nitrogen, or sulfur. Useful heteroaryl groups include piperazinyl, morpholinyl, piperidinyl, pyrrolidinyl, thienyl, furyl, pyranyl, pyrrolyl, imidazolyl, pyrazolyl, pyridinyl, including but not limited to pyrimidinyl, etc. The heteroaryl group may be optionally substituted with 1-5 (for example, 1, 2, 3, 4 or 5) substituents selected from the following group: a halogen, C₁₋₄ aldehyde group, C₁₋₆ straight-chain or branched alkyl, cyano, nitro, amino, hydroxy, hydroxymethyl, halogen-substituted alkyl (e.g., trifluoromethyl), halogen-substituted alkoxy (e.g., trifluoromethoxy), carboxyl, C₁₋₄ alkoxy, ethoxycarbonyl, N(CH₃), and C₁₋₄ acyl.

As used herein, "alkoxy" refers to an oxy group substituted by an alkyl. A preferred alkoxy is one having 1-6, and more preferably 1-3 carbon atoms. Examples of alkoxy include, but are not limited to, methoxy, ethoxy, propoxy, etc. The alkoxy may be substituted by one or more substituents, such as a halogen or haloalkyl. For example, the alkoxy may be an alkyl substituted by 1-4 fluorine atoms, or the alkyl may be an alkyl substituted by a fluoroalkyl.

As used herein, "halogen" refers to fluorine, chlorine, bromine, or iodine.

Based on the teachings of the present invention and common general knowledge in the art, a skilled person will understand that various groups in the compounds of the present invention can be further substituted, thereby obtaining derivatives that have the same or similar activity as the compounds specifically disclosed in the present invention. Various groups in the compounds of the present invention can be substituted by various conventional substituents in the art, as long as such substitution does not violate chemical synthesis rules or valence rules.

The term "substituted" as used herein refers to the replacement of one or more hydrogen atoms on a specific group with a specific substituent. The specific substituent can be the substituent described above, or it can be a specific substituent that appears in each Example or a conventional substituent in the field. Therefore, in this invention, the substituents in the general formula can also independently be the corresponding groups in the specific compounds of the Examples; that is, not only the combinations of substituents in the above general formula but also the combinations of some substituents shown in the general formula with other specific substituents that appear in the Examples will be included in this invention. It is readily for a skilled person to prepare compounds with such combinations of substituents and test the activity of the resulting compounds based on the routine technical means in the field. In other words, a skilled person can, based on the teachings of this invention, synthesize various compounds that fall within the protection scope of this invention, which are not limited to the specific compounds disclosed in the Examples of the specification; and the compounds of this invention include both the specific compounds disclosed in the Examples and various compounds formed by the specific substituent at a substitution position in these specific compounds with substituents at other substitution positions in the general formula. Such compounds will not be described one by one due to the limited contents.

As used herein, "optionally substituted" means that the modified substituent may be optionally substituted with 1-5 (for example, 1, 2, 3, 4 or 5) substituents selected from the following group: a halogen, C₁₋₄ aldehyde group, C₁₋₆ straight-chain or branched alkyl, cyano, nitro, amino, hydroxyl, hydroxymethyl, halogen-substituted alkyl (e.g., trifluoromethyl), halogen-substituted alkoxy (e.g., trifluoromethoxy), carboxyl, C₁₋₄ alkoxy, ethoxycarbonyl, N(CH₃) and C₁₋₄ acyl.

### The compounds of the present invention

The inventors synthesized candidate compounds with RSK-inhibiting activity. Structural optimization was carried out on the obtained candidate compounds, and a series of pyridonopyrimidine compounds not reported in the literature were designed and synthesized, and subjected to structural characterization. Activity tests at the molecular level were carried out on this series of compounds, and a batch of compounds capable of inhibiting RSK kinase activity were obtained.

The compound of the present invention is a compound shown in Formula I or a pharmaceutically acceptable salt thereof: 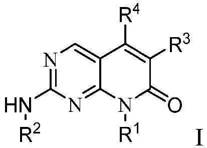 wherein R¹, R², R³ and R⁴ are described as above.

Further, the compound of the present invention can be a compound shown in Formula II or a pharmaceutically acceptable salt thereof: 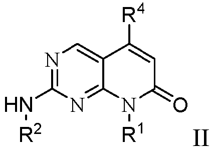 wherein R¹, R² and R⁴ are described as above.

In a specific embodiment, a series of pyridonopyrimidine compounds which have structures not reported in the literature are provided in the present invention. The specific compounds are as follows:

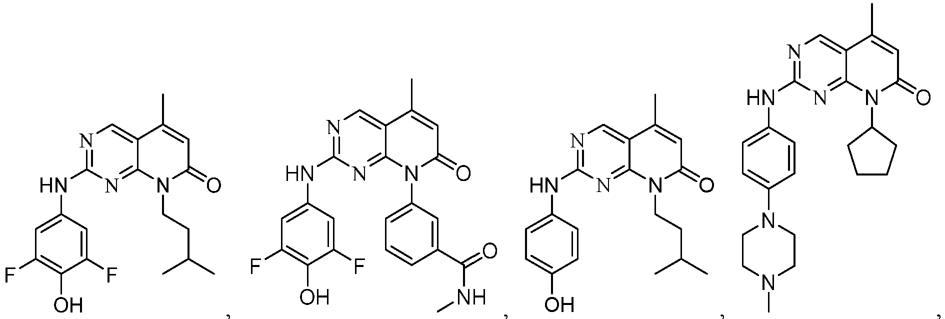

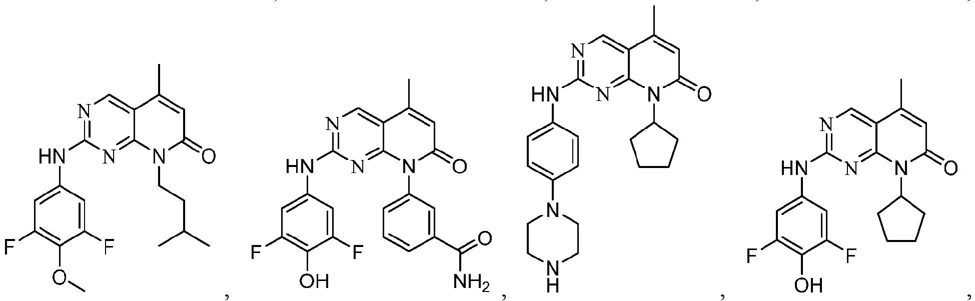

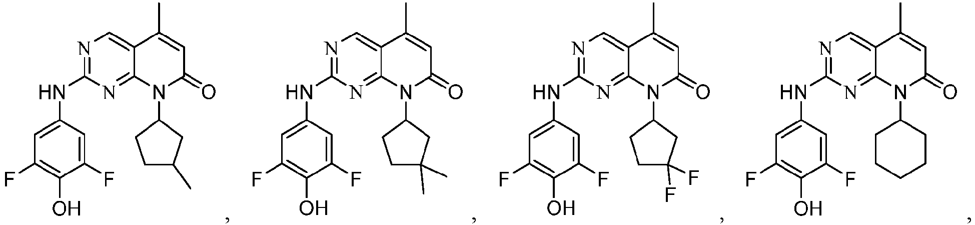

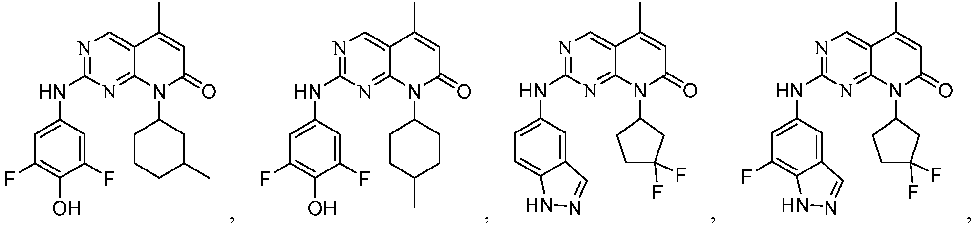

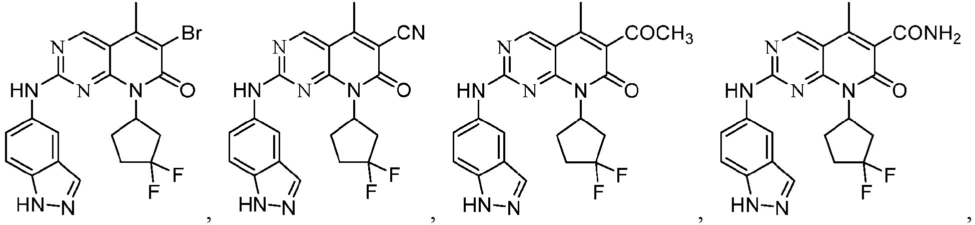

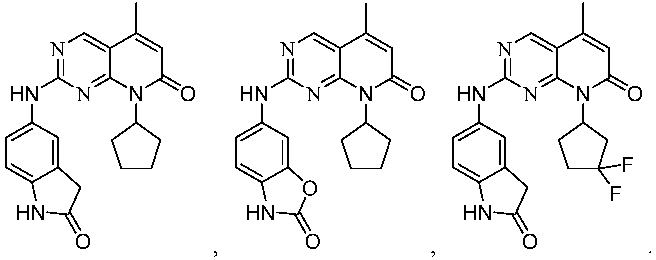

A skilled person will understand that the compounds of the present invention should also include all pharmaceutically acceptable isotopically labeled compounds, wherein one or more atoms are replaced by atoms having the same number of atoms but a different atomic mass or mass number than the atoms commonly found in nature. Isotopes suitable for inclusion in the compounds of the present invention include isotopes of hydrogen, such as ²H and ³H, isotopes of carbon, such as ¹¹C, ¹³C, and ¹⁴C, isotopes of nitrogen, such as ¹³N and ¹⁵N, and isotopes of oxygen, such as ¹⁵O, ¹⁷O, and ¹⁸O.

Substitution with heavier isotopes, such as deuterium (² H), may provide certain therapeutic advantages, such as better metabolic stability. For example, an increased half-life *in vivo* or reduced dosage requirement may be preferred in certain situations.

Based on the teachings of the present invention, a skilled person will understand that the compounds of the present invention are pharmaceutically active compounds, and thus can be used as medicaments. As pharmaceutically active compounds, the compounds of the present invention naturally should also possess various inherent characteristics of medicaments, such as druggability, bioavailability, low toxicity and side effects, etc. Based on the teachings of the present invention, a skilled person can employ various well-known existing technical means to obtain the compounds of the present invention and test the above-mentioned characteristics; in other words, based on the teachings of the present invention, a skilled person can repeat or verify the present invention.

Based on the compounds of the present invention, a pharmaceutical composition comprising a therapeutically effective amount of the compound of the present invention or a pharmaceutically acceptable salt, and a pharmaceutically acceptable carrier or excipient is provided in the present invention.

Examples of the pharmaceutically acceptable salt of the compounds of the present invention include, but are not limited to, inorganic and organic acid salts, such as hydrochloride, hydrobromide, sulfate, citrate, lactate, tartrate, maleate, fumarate, mandelate and oxalate; and inorganic and organic base salts formed with bases such as sodium hydroxide, tris(hydroxymethyl)aminomethane (TRIS, trometamol) and N - methylglucamine.

The pharmaceutical composition of the present invention can be formulated into dosage forms suitable for various administration routes, including but not limited to those for parenteral, subcutaneous, intravenous, intramuscular, intraperitoneal, transdermal, oral, intrathecal, intracranial, nasal or topical administration, for treating tumors and other diseases. The dosage is an amount of the medicament that is effective in improving or eliminating one or more conditions. For the treatment of a specific disease, the effective amount is an amount of the medicament that is sufficient to improve or alleviate symptoms associated with the disease in some way. Such an amount of the medicament may be administered as a single dose, or may be administered according to an effective treatment regimen. The dosage may cure the disease, but generally be for improving the symptoms of the disease. Generally, repeated dosing is required to achieve the desired improvement of symptoms. The dosage of the medicament will be determined based on the patient's age, health and weight, the type of concomitant therapy, the frequency of treatment, and the desired therapeutic benefits.

The pharmaceutical formulation of the present invention can be administered to any mammal, as long as the therapeutic effects of the compounds of the present invention can be achieved in them. Among these mammals, humans are the most important.

The compound of the present invention or a pharmaceutical composition thereof can be used to treat various diseases mediated by RSK protein kinases. Herein, the diseases mediated by RSK protein kinases are various cancers. The cancers include, but are not limited to: breast cancer regulated by RSK1 and RSK4, ovarian cancer regulated by RSK3 and RSK4, prostate cancer regulated by RSK1 and RSK2, lung cancer regulated by RSK1, RSK2 and RSK4, head and neck squamous cell carcinoma and acute myeloid leukemia regulated by RSK2, esophageal cancer, renal cancer, endometrial cancer, colon cancer and other cancers and solid tumors regulated by RSK4.

The pharmaceutical formulation of the present invention can be manufactured in a known manner. For example, it can be manufactured by traditional processes such as mixing, granulation, tabletting, dissolution, or freeze-drying. When manufacturing an oral formulation, a solid excipient and the active compound can be combined, and the mixture can be selectively ground. After adding an appropriate amount of auxiliary agent, the granular mixture is processed to obtain a tablet or tablet core, if needed or necessary.

Appropriate excipients, especially fillers, such as sugars like lactose or sucrose, mannitol or sorbitol; cellulose preparations or calcium phosphates, for example, tricalcium phosphate or calcium hydrogen phosphate; as well as binders, such as starch paste, including corn starch, wheat starch, rice starch, potato starch, gelatin, tragacanth gum, methylcellulose, hydroxypropyl methylcellulose, sodium carboxymethyl cellulose, or polyvinylpyrrolidone. If necessary, disintegrants can be added, such as the starches mentioned above, as well as carboxymethyl starch, cross-linked polyvinylpyrrolidone, agar, or alginic acid or its salts, such as sodium alginate. Auxiliary agents, especially flow regulators and lubricants, for example, silica, talc, stearates such as calcium stearate, magnesium stearate, stearic acid, or polyethylene glycol. If necessary, a suitable coating that is resistant to gastric juice can be provided for the tablet core. For this purpose, concentrated sugar solutions can be used. This solution can contain gum arabic, talc, polyvinylpyrrolidone, polyethylene glycol and/or titanium dioxide, varnish solutions, and appropriate organic solvents or solvent mixtures. To prepare a gastric juice-resistant coating, an appropriate cellulose solution can be used, such as cellulose acetate phthalate or hydroxypropyl methylcellulose phthalate. Dyes or pigments can be added to the coating of the tablet or tablet core for, for example, the identification or characterizing the combination of active ingredient dosages.

Based on the above compounds and pharmaceutical compositions, a method for treating diseases mediated by RSK protein kinases is further provided in the present invention, comprising administering to a subject in need thereof the compound or pharmaceutical composition of the present invention.

The administration methods include, but are not limited to, various administration methods well known in the art, which can be determined according to the actual situation of a patient. Such methods include, but are not limited to, the administration via parenteral, subcutaneous, intravenous, intramuscular, intraperitoneal, transdermal, oral, intrathecal, intracranial, nasal or topical routes.

The use of the compounds of the present invention in the preparation of a medicament for preventing or treating RSK-mediated diseases or inhibiting the activity of RSK4 is also included in the present invention.

### Advantages of the present invention:

1. The compound provided in the present invention is a pyridonopyrimidine compound with a completely novel structure;
2. The compound provided in the present invention exhibits excellent inhibitory activity against RSK protein kinases;
3. The compound provided in the present invention lays a foundation for the development of medicaments capable of inhibiting RSK-targeted pathways, showing great potential for industrialization and commercialization as well as market value with significant economic benefits.

The technical solutions of the present invention will be further described below in connection with specific embodiments, however, following embodiments do not constitute a limitation of the present invention, and all the various embodiments based on the principles and technical means of the present invention shall fall within the scope of the present invention. Experimental methods for which specific conditions are not indicated in the following embodiments are generally in accordance with conventional conditions, or in accordance with conditions recommended by the manufacturer. Percentages and portions are calculated by weight unless otherwise indicated.

### Material and method

### The synthesis of the pyridonopyrimidine compounds of the present invention is shown as follows:

### Example 1

### 2-((1H-indazol-5-yl)amino)-8-cyclopentyl-5-methylpyrido[2,3-d]pyrimidin-7(8H)-one (001)

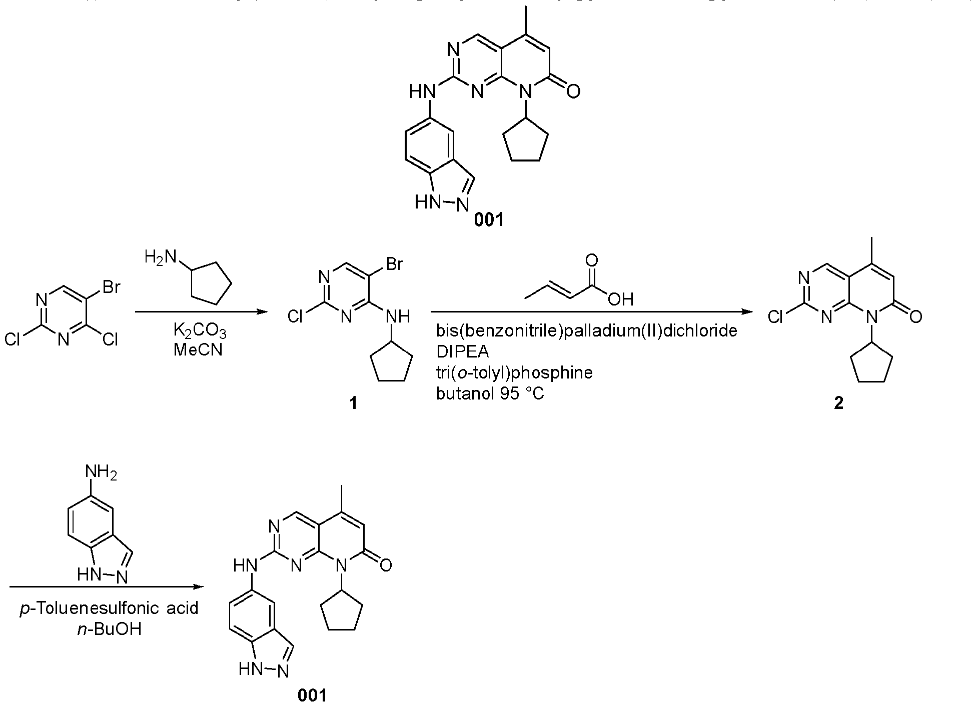

### Step 1 5-Bromo-2-chloro-N-cyclopentylpyrimidin-4-amine (1)

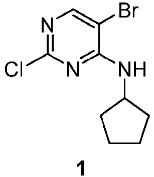

5-bromo-2,4-dichloropyrimidine (3.2 g, 14.2 mmol) and cyclopentylamine (1.2 g, 14.2 mmol) were added successively into a 100 mL single-necked round-bottom flask, and then 15 mL of acetonitrile was added as the solvent. After cooled to 0 °C in an ice-salt bath, K₂CO₃ (3.9 g, 28.4 mmol) was added, stirred for 10 min under an ice bath, then warmed up to the room temperature and stirred for 10 h. The reaction was monitored by thin-layer chromatography (TLC). After the reaction was completed, 100 mL of water was added, and extracted with ethyl acetate. The organic layers were combined, and the organic phase was washed with a saturated sodium chloride solution and dried over anhydrous sodium sulfate. The solvent was removed under a reduced-pressure to obtain a white oily liquid, which was purified by rapid silica gel column chromatography (petroleum ether/ethyl acetate = 30: 1) to obtain the corresponding white solids, 5-bromo-2-chloro-*N*-cyclopentylpyrimidin-4-amine, 2.6 g, with a yield of 66%.

¹H NMR (400 MHz, DMSO- *d*6) δ 9.00 (s, 1H), 8.61 (d, J = 7.6 Hz, 1H), 4.53 - 4.44 (m, 1H), 2.00 - 1.95 (m, 2H), 1.75 - 1.69 (m, 2H), 1.67 - 1.62 (m, 2H), 1.60 - 1.56 (m, 2H). LC-MS: m/z: 275.3 (M+H)⁺.

### Step 2 2-Chloro-8-cyclopentyl-5-methylpyrido[2,3-d]pyrimidin-7(8H)-one (2)

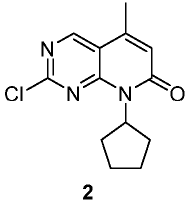

In a 50 mL two-necked round-bottom flask, 5-bromo-2-chloro-*N*-cyclopentylpyrimidin-4-amine (1.5 g, 5.5 mmol), butenoic acid (2.3 g, 27.5 mmol), bis(benzonitrile)palladium chloride (230 mg, 0.6 mmol), tris(o-tolyl)phosphine (183 mg, 0.6 mmol), N,N-diisopropylethylamine (7.2 g, 55 mmol) and n-butanol (6 mL) were added successively, and the flask was purged with nitrogen for three times. The reaction was carried out at 95 °C for 4 h, and monitored by TLC. After the reaction was completed, the mixture was directly concentrated under a reduced pressure, and separated and purified by flash silica gel column chromatography (dichloromethane/methanol = 10:1) to obtain a reddish-brown oily liquid. To the reddish-brown oily liquid, 10 mL of acetic anhydride was added, and the reaction was carried out at 130 °C for 1 h and monitored by TLC. After the reaction was completed, the solvent was directly removed by vacuum rotary evaporation, and the product was separated and purified by flash silica gel column chromatography (dichloromethane/methanol = 300:1) to obtain 434 mg of intermediate **2** as pale yellow solids with a yield of 30%

¹H NMR (400 MHz, DMSO-*d₆*) δ 8.29 (s, 1H), 7.72 (s, 1H), 4.63-4.49 (m, 1H), 2.51 (s, 3H), 2.44 - 2.19 (m, 2H), 2.17-2.10 (m, 3H), 2.00 - 1.86 (m, 3H). LC-MS: m/z: 264.1 (M+H)⁺.

### Step 3 2-((1H-indazol-5-yl)amino)-8-cyclopentyl-5-methylpyrido[2,3-d]pyrimidin-7(8H)-one (001)

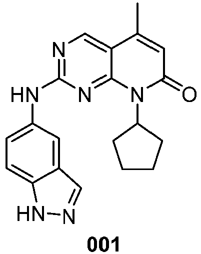

In a 100 mL single-necked round-bottom flask, 2-chloro-8-cyclopentyl-5-methylpyrido[2,3-d]pyrimidin-7(8*H*)-one (400 mg, 1.5 mmol), 5-aminoindazole (202 mg, 1.5 mmol), p-toluenesulfonic acid (261 mg, 1.5 mmol), and n-butanol (4 mL) were added sequentially. The reaction was carried out at 95 °C for 12 hours and monitored by TLC. After the reaction was completed, 100 mL of saturated NaCl aqueous solution was added, and the mixture was extracted with ethyl acetate for three times. The organic layers were combined and dried over anhydrous sodium sulfate, and the solvent was removed under a reduced pressure. Column chromatography (dichloromethane/methanol = 30:1) was employed to isolate the corresponding compound 001, pale yellow solids (145 mg), with a yield of 27%.

¹H NMR (400 MHz, DMSO-*d₆*) δ 13.00 (s, 1H), 9.94 (s, 1H), 8.81 (s, 1H), 8.12 (s, 1H), 8.00 (s, 1H), 7.53 (q, *J =* 8.7 Hz, 2H), 6.18 (s, 1H), 5.84-5.80 (m, 1H), 2.37 (s, 3H), 2.25-2.20 (m, 2H), 1.88 - 1.69 (m, 4H), 1.56-1.50 (m, 2H). ¹³C NMR (151 MHz, DMSO-*d₆*) δ 163.01, 159.72, 157.27, 156.04, 145.83, 137.32, 133.56, 132.98, 123.33, 122.52, 117.61, 111.20, 110.42, 107.20, 53.01, 27.95, 25.45, 17.15. HRMS (ESI) (m/z): [M+H]⁺calcd for C₂₀H₂₀N₆O, 361.1780; found 361.1779.

### N-(8-Cyclopentyl-5-methyl-7-oxo-7,8-dihydropyrido[2,3-d]pyrimidin-2-yl)-N-(3,5-difluoro-4-hydroxyphenyl)acetamide (002)

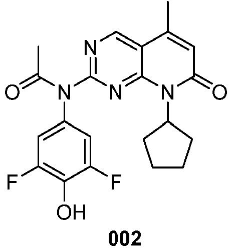

Synthesis method is described as in Example 1.

### 8-Cyclopentyl-2-((3,5-difluoro-4-hydroxyphenyl)amino)-5-methylpyrido[2,3-d]pyrimidin-7( 8H)-one (003)

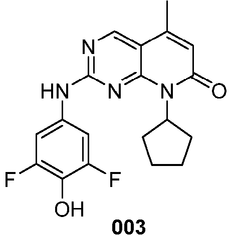

The synthesis method is described as in Example 1.

Light yellow solids, with a yield of 35%.

¹H NMR (400 MHz, DMSO-d6) δ 10.01 (s, 1H), 9.77 (s, 1H), 8.83 (s, 1H), 7.44 (d, J = 10.0 Hz, 2H), 6.21 (s, 1H), 5.89 - 5.69 (m, 1H), 2.37 (s, 3H), 2.25-2.20 (m, 2H), 1.93-1.80 (m, 2H), 1.83 - 1.69 (m, 2H), 1.67 - 1.53 (m, 2H). ¹³C NMR (151 MHz, DMSO-*d*6) δ 162.88, 158.92, 157.23, 155.94, 153.34, 153.28, 151.76, 151.70, 145.77, 131.70, 129.02, 128.92, 118.33, 107.66, 104.04, 103.99, 103.86, 27.93, 25.45, 17.13. HRMS (ESI) (m/z): [M+H]⁺ calcd for C₁₉H₁₈F₂N₄O₂, 373.1476; found 373.1475.

### 2-((1H-benzo[d][1,2,3]triazol-5-yl)amino)-8-cyclopentyl-5-methylpyrido[2,3-d]pyrimidin-7( 8H)-one (004)

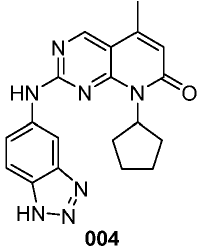

The synthesis method is described as in Example 1.

Light yellow solids, with a yield of 35%.

¹H NMR (400 MHz, DMSO-*d₆*) δ 15.56 (s, 1H), 10.26 (s, 1H), 8.89 (s, 1H), 8.33 (s, 1H), 7.95 (s, 1H), 7.63 (s, 1H), 6.24 (s, 1H), 6.00 - 5.76 (m, 1H), 2.40 (s, 3H), 2.25-2.20 (m, 2H), 1.95-1.90 (m, 2H), 1.82-1.78 (m, 2H), 1.70 - 1.56 (m, 2H). ¹³C NMR (151 MHz, DMSO-*d₆*) δ 163.11, 159.67, 157.56, 155.76, 146.56, 137.32, 133.60, 132.85, 123.33, 122.36, 117.41, 111.08, 110.48, 60.83, 35.16, 19.11, 17.21, 14.33. HRMS (ESI) (m/z): [M+H]⁺ calcd for C₁₉H₁₉N₇O, 362.1730; found 362.1731.

### 2-((1H-indazol-5-yl)amino)-8-(3,3-difluorocyclopentyl)-5-methylpyrido[2,3-d]pyrimidin-7(8 H)-one (005)

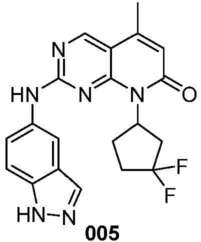

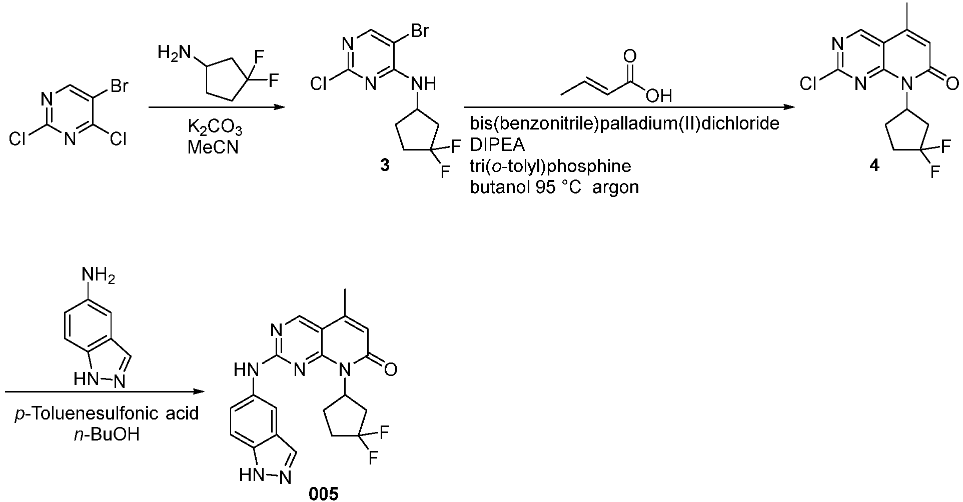

### Step 1 5-Bromo-2-chloro-N-(3,3-difluorocyclopentyl)pyrimidin-4-amine (3)

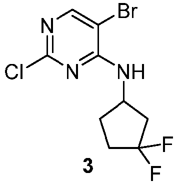

In a 100 mL single-necked round-bottom flask, 5-bromo-2,4-dichloropyrimidine (2.0 g, 8.9 mmol) and 3,3-difluorocyclopentylamine hydrochloride (1.4 g, 8.9 mmol) were added successively, and then 15 mL of acetonitrile was added as the solvent. After cooled to 0 °C in an ice-salt bath, K₂CO₃ (2.5 g, 17.8 mmol) was added. The mixture was stirred at 0 °C for 10 min in an ice bath, then allowed to warm up to the room temperature and stirred for 10 h. The reaction was monitored by TLC. After the reaction was completed, 100 mL of water was added, and the mixture was extracted with ethyl acetate for three times. The organic layers were combined, washed with a saturated sodium chloride solution and dried over anhydrous sodium sulfate, and the solvent was removed under a reduced pressure to obtain a white oily liquid, which was purified by rapid silica gel column chromatography (petroleum ether/ethyl acetate = 30: 1) to yield 1.9 g of the corresponding white solids, 5-bromo-2-chloro-N-(3,3-difluorocyclopentyl)pyrimidin-4-amine, with a yield of 70%.

### Step 2 2-Chloro-8-(3,3-difluorocyclopentyl)-5-methylpyrido[2,3-d]pyrimidin-7(8H)-one (4)

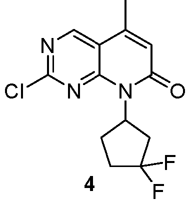

In a 50 mL two-necked round-bottom flask, 5-bromo-2-chloro-*N*-cyclopentylpyrimidin-4-amine (1.5 g, 5 mmol), butenoic acid (2.2 g, 25 mmol), bis(benzonitrile)palladium chloride (191 mg, 0.5 mmol), tris(o-tolyl)phosphine (152 mg, 0.5 mmol), N,N-diisopropylethylamine (6.5 g, 50 mmol), and n-butanol (6 mL) were added sequentially. The flask was purged with nitrogen for three times. The reaction was carried out at 95 °C for 4 h, and monitored by TLC. After the reaction was completed, the mixture was directly concentrated under a reduced pressure, and purified by flash silica gel column chromatography (dichloromethane/methanol = 10:1) to obtain a reddish-brown oily liquid. To the reddish-brown oily liquid, 10 mL of acetic anhydride was added, and the mixture was reacted at 130 °C for 1 h, and monitored by TLC. After the reaction was completed, the solvent was directly removed under a reduced pressure, and the product was purified by flash silica gel column chromatography (dichloromethane/methanol = 300:1) to obtain 2-chloro-8-(3,3-difluorocyclopentyl)-5-methylpyrido[2,3-d]pyrimidin-7(8*H*)-one as pale yellow solids (449 mg), with a yield of 30%.

¹H NMR (400 MHz, DMSO-*d₆*) δ 9.07 (s, 1H), 6.64 (s, 1H), 5.99-5.83 (m, 1H), 2.96-2.85 (m, 1H), 2.65 (m, 1H), 2.46 (s, 3H), 2.43 - 2.31 (m, 2H), 2.28 - 2.08 (m, 2H). LC-MS: m/z: 300.2 (M+H)⁺.

### Step 3 2-((1H-indazol-5-yl)amino)-8-(3,3-difluorocyclopentyl)-5-methylpyrido[2,3-d]pyrimidin-7(8H)-on e (005)

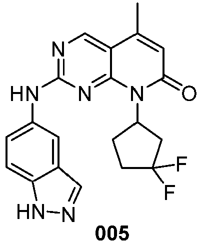

2-chloro-8-(3,3-difluorocyclopentyl)-5-methylpyrido[2,3-d]pyrimidin-7(8H)-one (400 mg, 1.3 mmol), 5-aminoindazole (178 mg, 1.3 mmol), p-toluenesulfonic acid (224 mg, 1.3 mmol) and 4 mL of n-butanol were added successively into a 100 mL single-necked round-bottom flask. The reaction was carried out at 95 °C for 12 h, and monitored by TLC. After the reaction was completed, 100 mL of saturated NaCl aqueous solution was added, and extracted with ethyl acetate. the organic layers were combined and dried over anhydrous sodium sulfate, and the solvent was removed under a reduced pressure. The residue was separated by column chromatography (dichloromethane/methanol = 30:1) to obtain **005,** pale yellow solids (180 mg) with a yield of 35%.

¹H NMR (600 MHz, DMSO-*d₆*) *δ* (ppm): 13.00 (s, 1H), 10.05 (s, 1H), 8.85 (s, 1H), 8.17 - 8.12 (m, 1H), 7.97 (s, 1H), 7.56 (dd, *J* = 8.9, 1.9 Hz, 1H), 7.50 (d, *J* = 8.9 Hz, 1H), 6.21 (d, *J* = 1.4 Hz, 1H), 6.11 (s, 1H), 3.03 (dq, *J* = 27.6, 11.9 Hz, 1H), 2.49 - 2.41 (m, 2H), 2.39 (d, *J* = 1.2 Hz, 3H), 2.28 (ddt, *J =* 19.0, 13.2, 6.5 Hz, 1H), 2.12 - 1.96 (m, 2H). LC-MS: m/z calc. for C₂₀H₁₈F₂N₆O [M+H]⁺: 397.15, found 397.20.

### 2-((1H-indazol-5-yl)amino)-5-methyl-8-(pyrrolidin-3-ylamino)pyrido[2,3-d]pyrimidin-7(8H) -one (006)

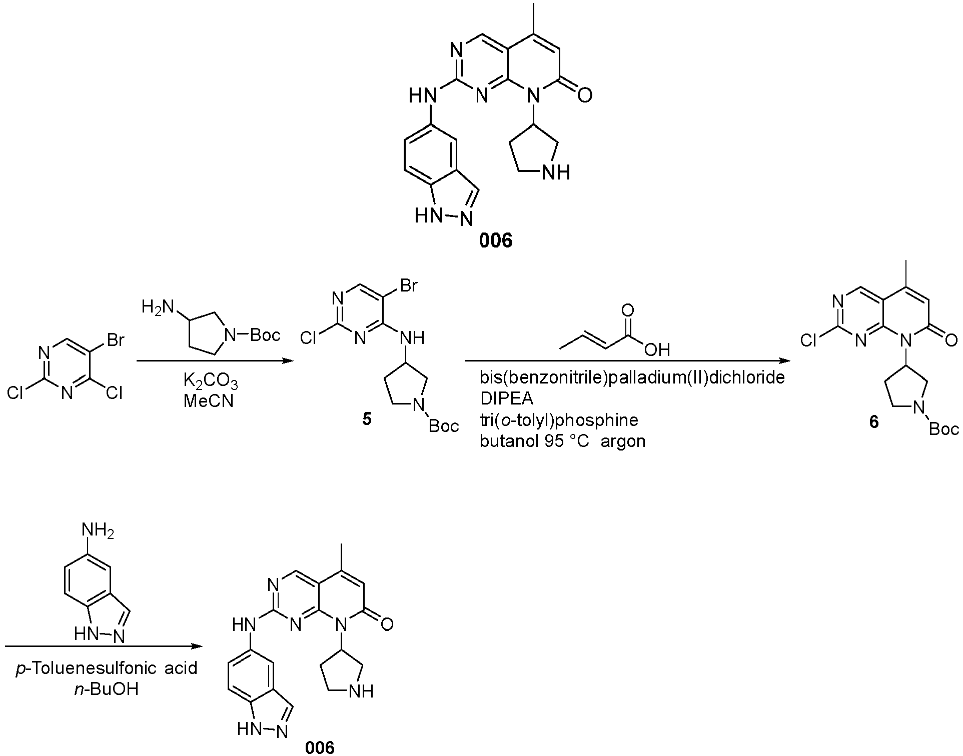

### Step 1 tert-Butyl 3-((5-bromo-2-chloropyrimidin-4-yl)amino)pyrrolidine-1-carboxylate (5)

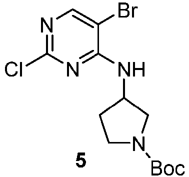

5-bromo-2,4-dichloropyrimidine (2.5 g, 11.1 mmol) and tert-butyl 3-aminopyrrolidine-1-carboxylate (2.1 g, 11.1 mmol) were added successively into a 100 mL single-necked round-bottom flask, and then 15 mL of acetonitrile was added as the solvent. After cooled to 0 °C in an ice-salt bath, K₂CO₃ (3.1 g, 22.2 mmol) was added and stirred for 10 min under an ice bath, then warmed up to room temperature and stirred for 10 h. The reaction was monitored by TLC. After the reaction was completed, 100 mL of water was added and extracted with ethyl acetate for three times. The organic layers were combined and the organic phase was washed with a saturated sodium chloride solution, and dried over anhydrous sodium sulfate. The solvent was removed under a reduced pressure to obtain a white oily liquid, which was purified by rapid silica gel column chromatography (petroleum ether/ethyl acetate = 30:1) to obtain the corresponding tert-butyl 3-(5-bromo-2-chloropyrimidin-4-ylamino)pyrrolidine-1-carboxylate, white solids, 2.1 g, with a yield of 50%.

¹H NMR (400 MHz, DMSO-*d₆*) δ 8.30 (s, 1H), 7.64 (d, *J =* 6.9 Hz, 1H), 4.74 - 4.34 (m, 1H), 3.58 (dd, *J =* 10.8, 7.3 Hz, 1H), 3.46 - 3.36 (m, 1H), 3.31 - 3.09 (m, 2H), 2.19 - 1.85 (m, 2H) , 1.41 (s, 9H).

### Step 2 tert-Butyl 3-(2-chloro-5-methyl-7-oxopyrido[2,3-d]pyrimidin-8(7H)-yl)pyrrolidine-1-carboxylate (6)

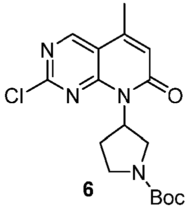

In a 50 mL two-necked round-bottom flask, 5-bromo-2-chloro-*N*-cyclopentylpyrimidin-4-amine (1.5 g, 4.0 mmol), butenoic acid (1.7 g, 20 mmol), bis(benzonitrile)palladium chloride (153 mg, 0.4 mmol), tris(o-tolyl)phosphine (122 mg, 0.4 mmol), N,N-diisopropylethylamine (5.1 g, 40 mmol), and n-butanol (6 mL) were added sequentially. The flask was purged with nitrogen for three times. The reaction was carried out at 95 °C for 4 h, and monitored by TLC. After the reaction was completed, the mixture was directly concentrated under a reduced pressure, and purified by flash silica gel column chromatography (dichloromethane/methanol = 10:1) to obtain a reddish-brown oily liquid. To the reddish-brown oily liquid, 10 mL of acetic anhydride was added, and the mixture was reacted at 130 °C for 1 h and monitored by TLC. After the reaction was completed, the solvent was directly removed *in vacum,* and the residue was purified by flash silica gel column chromatography (dichloromethane/methanol = 300:1) to obtain tert-butyl 3-(2-chloro-5-methyl-7-oxopyrido[2,3-d]pyrimidin-8(7H)-yl)pyrrolidine-1-carboxylate as pale yellow solids (145 mg), with a yield of 10%.

¹H NMR (400 MHz, DMSO-*d₆*) δ 9.06 (s, 1H), 6.65 (s, 1H), 6.13 - 5.79 (m, 1H), 3.84 - 3.50 (m, 3H), 2.46 (s, 3H), 2.22-2.20 (m, 1H), 1.87 - 1.78 (m, 1H), 1.41 (d, *J =* 13.0 Hz, 9H).

### Step 3 2-((1H-indazol-5-yl)amino)-5-methyl-8-(pyrrolidin-3-ylamino)pyrido[2,3-d]pyrimidin-7(8H)-one (006)

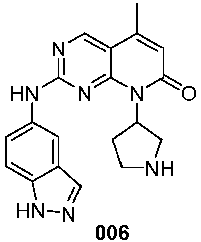

Tert-butyl 3-(2-chloro-5-methyl-7-oxopyrido[2,3-d]pyrimidin-8(7*H*)-yl)piperidine-1-carboxylate (200 mg, 0.6 mmol), 5-aminoindazole (80 mg, 0.6 mmol), p-toluenesulfonic acid (103 mg, 0.6 mmol) and 4 mL of n-butanol were added successively into a 100 mL single-necked round-bottom flask. The reaction was carried out at 95 °C for 12 h and monitored by TLC. After the reaction was completed, 100 mL of saturated sodium chloride aqueous solution was added and extracted with ethyl acetate. The organic layers were combined and dried over anhydrous sodium sulfate. And then the solvent was removed under a reduced pressure. The residue was separate by column chromatography (dichloromethane/methanol = 30:1) to obtain **006,** pale yellow solids, 43 mg, with a yield of 20%.

¹H NMR (400 MHz, DMSO-*d₆*) δ 13.05 (s, 1H), 9.95 (s, 1H), 8.82 (s, 1H), 8.10 (s, 1H), 8.04 (s, 1H), 7.61 - 7.43 (m, 2H), 6.20 (s, 1H), 5.96-5.90 (m, 1H), 3.17 (s, 1H), 3.09 (dd, *J* = 11.4, 5.9 Hz, 2H), 2.90 - 2.80 (m, 1H), 2.69-2.63 (m, 1H), 2.38 (s, 3H), 2.11-2.06 (m, 1H), 2.03 - 1.94 (m, 1H). ¹³C NMR (151 MHz, DMSO-*d₆*) δ 163.09, 163.07, 159.71, 157.37, 155.96, 146.05, 146.02, 132.90, 128.50, 128.48, 125.97, 125.95, 122.45, 122.42, 117.43, 111.26, 49.89, 48.39, 30.24, 29.49, 27.02, 21.25, 17.18, 0.58. HRMS (ESI) (m/z): [M+H]⁺ calcd for C₁₉H₁₉N₇O, 362.1729; found 362.1727.

### 8-Cyclopentyl-5-methyl-2-((2-oxoindolin-5-yl)amino)pyrido[2,3-d]pyrimidin-7(8H)-one (007)

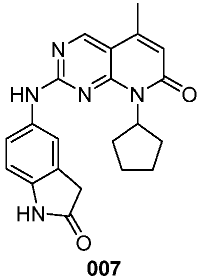

The synthesis method is described as in Example 1.

Light yellow solids, with a yield of 39%.

¹H NMR (400 MHz, DMSO-*d₆*) δ 10.33 (s, 1H), 9.83 (s, 1H), 8.74 (s, 1H), 7.61 (s, 1H), 7.43 (d, *J* = 8.4 Hz, 1H), 6.75 (d, *J* = 8.0 Hz, 1H), 6.16 (s, 1H), 5.81 (s, 1H), 3.43 (s, 2H), 2.30 (s, 3H), 2.34 - 2.14 (m, 2H), 1.92-1.90 (m, 2H), 1.77 - 1.64 (m, 2H), 1.65 - 1.51 (m, 2H). ¹³C NMR (151 MHz, DMSO-*d₆*) δ 176.69, 162.98, 159.47, 157.23, 156.02, 145.82, 139.52, 134.08, 126.41, 120.27, 118.14, 117.62, 109.25, 36.53, 27.98, 25.54, 17.13. HRMS (ESI) (m/z): [M+H]⁺ calcd for C₂₁H₂₁N₅O₂, 376.1773; found 376.1772.

### 6-((8-Cyclopentyl-5-methyl-7-oxo-7,8-dihydropyrido[2,3-d]pyrimidin-2-yl)amino)benzo[d]o xazol-2(3H)-one (008)

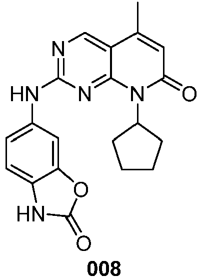

The synthesis method is described as in Example 1.

Light yellow solids, with a yield of 39%.

¹H NMR (400 MHz, DMSO-*d₆*) δ 9.91 (s, 1H), 8.80 (s, 1H), 7.48 (s, 1H), 7.27 (dd, *J* = 8.6, 1.9 Hz, 1H), 7.17 (d, *J* = 8.6 Hz, 1H), 6.19 (s, 1H), 5.87 - 5.75 (m, 1H), 2.37 (s, 3H), 2.18-2.00 (m, 2H), 1.88-1.80 (m, 2H), 1.80 - 1.70 (m, 2H), 1.63 - 1.52 (m, 2H).¹³C NMR (151 MHz, DMSO-*d₆*) δ 162.92, 159.35, 157.21, 156.02, 156.59, 145.78, 139.99, 135.93, 130.41, 117.95, 113.52, 109.14, 107.24, 103.09, 52.94, 28.17, 25.72, 17.15. HRMS (ESI) (m/z): [M+H]⁺calcd for C₂₀H₁₉N₅O₃, 378.1566; found 378.1565.

### 8-Cyclopentyl-2-((7-fluoro-1H-indazol-5-yl)amino)-5-methylpyrido[2,3-d]pyrimidin-7(8H)-one (009)

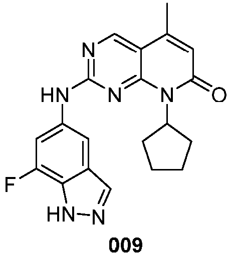

The synthesis method is described as in Example 1.

Light yellow solids, with a yield of 39%.

¹H NMR (400 MHz, DMSO-*d₆*) δ 13.56 (s, 1H), 10.07 (s, 1H), 8.84 (s, 1H), 8.12 (s, 1H), 7.91 (s, 1H), 7.59 (d, *J =* 13.2 Hz, 1H), 6.20 (s, 1H), 5.97 - 5.73 (m, 1H), 2.38 (s, 3H), 2.26-2.20 (m, 2H), 1.88-1.80 (m, 2H), 1.78-1.70 (m, 3H), 1.58-1.52 (m, 2H). ¹³C NMR (151 MHz, DMSO-*d₆*) δ 162.97, 159.45, 157.30, 156.00, 148.01, 146.39, 145.83, 134.40, 133.63, 126.72, 126.64, 117.98, 107.51, 106.50, 53.16, 27.97, 25.44, 17.15. HRMS (ESI) (m/z): [M+H]⁺calcd for C₂₀H₁₉FN₆O, 379.1683; found 379.1681.

### 2-((1H-indazol-5-yl)amino)-6-bromo-8-cyclopentyl-5-methylpyrido[2,3-d]pyrimidin-7(8H)-one (011)

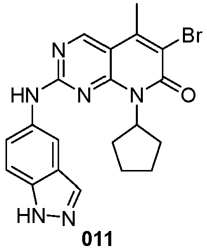

The synthesis method is described as in Example 1.

¹H NMR (400 MHz, DMSO-*d₆*) δ 13.02 (s, 1H), 10.10 (s, 1H), 8.99 (s, 1H), 8.08 (s, 1H), 8.00 (s, 1H), 7.54 (q, *J* = 8.7 Hz, 2H), 5.98 - 5.55 (m, 1H), 2.56 (s, 3H), 2.120 - 2.12 (m, 2H), 1.88 - 1.79 (m, 4H), 1.58 - 1.51 (m, 2H). ¹³C NMR (151 MHz, DMSO-*d₆*) δ 159.57, 158.57, 158.14, 154.67, 145.20, 142.9, 137.37, 133.61, 132.79, 123.32, 122.42, 114.8, 111.26, 110.47, 60.01, 28.01, 25.60, 18.36. HRMS (ESI) (m/z): [M+H]⁺ calcd for C₂₀H₁₉BrN₆O, 439.0882; found 439.0884.

### 8-Cyclopentyl-2-[(3-fluoro-1H-indazol-5-yl)amino]-5-methylpyrido[2,3-d]pyrimidin-7(8H)-one (012)

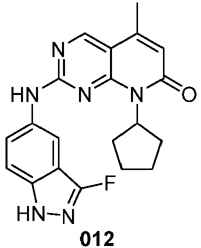

The synthesis method is described as in Example 1.

### 2-((1H-pyrazolo[3,4-b]pyridin-5-yl)amino)-8-cyclopentyl-5-methylpyrido[2,3-d]pyrimidin-7 (8H)-one (013)

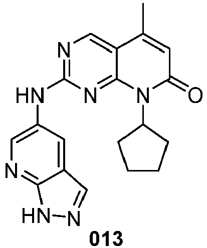

The synthesis method is described as in Example 1.

Light yellow solids, with a yield of 39%.

¹H NMR (400 MHz, DMSO-*d₆*) δ 13.61 (s, 1H), 10.06 (s, 1H), 8.83 (s, 1H), 8.71 (s, 1H), 8.46 (s, 1H), 8.10 (s, 1H), 6.21 (s, 1H), 5.88-5.70 (m, 1H), 2.38 (s, 3H), 2.332.19 (m, 2H), 1.79-1.69 (m, 4H), 1.59-1.50 (m, 2H). ¹³C NMR (151 MHz, DMSO-*d₆*) δ 162.97, 159.84, 157.40, 156.02, 149.22, 145.85, 145.31, 133.27, 130.50, 120.97, 117.89, 114.47, 108.60, 107.68, 52.94, 32.01, 27.96, 17.17. HRMS (ESI) (m/z): [M+H]⁺ calcd for C₁₉H₁₉N₇O, 362.1730; found 362.1730.

### 6-Bromo-8-cyclopentyl-5-methyl-2-(2-oxoindolin-5-ylamino)pyrido[2,3-d]pyrimidin-7(8H)-one (014)

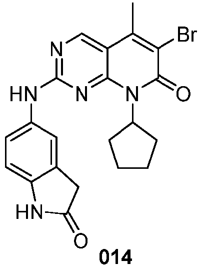

The synthesis method is described as in Example 1.

¹H NMR (400 MHz, DMSO-*d₆*) δ 10.33 (s, 1H), 9.98 (s, 1H), 8.93 (s, 1H), 7.61 (s, 1H), 7.43 (d, *J* = 7.7 Hz, 1H), 6.78 (d, *J =* 8.3 Hz, 1H), 5.99-5.89 (m, 1H), 3.48 (s, 2H), 2.55 (s, 3H), 2.25-2.10 (m, 2H), 1.99-1.89 (m, 2H), 1.84-1.74 (m, 2H), 1.65-1.59 (m, 2H). ¹³C NMR (151 MHz, DMSO-*d₆*) δ 176.69, 159.30, 158.51, 158.08, 154.65, 145.16, 139.66, 133.87, 130.12, 126.45, 120.26, 118.09, 114.93, 109.27, 60.83, 36.52, 28.03, 25.69, 18.33. HRMS (ESI) (m/z): [M+H]⁺ calcd for C₂₁H₂₀BrN₅O₂, 454.0876; found 454.0879.

### 8-(3,3-Difluorocyclopentyl)-5-methyl-2-((2-oxoindolin-5-yl)amino)pyrido[2,3-d]pyrimidin-7 (8H)-one (015)

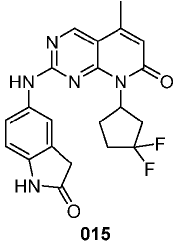

Light yellow solids, with a yield of 39%.

¹H NMR (400 MHz, DMSO-*d₆*) δ 10.33 (s, 1H), 9.93 (s, 1H), 8.81 (s, 1H), 7.60 (s, 1H), 7.42 (d, *J* = 8.2 Hz, 1H), 6.77 (d, *J =* 8.0 Hz, 1H), 6.17 (d, *J =* 16.0 Hz, 1H), 6.15-6.00 (m, 1H), 3.46 (s, 2H), 3.01 (td, *J =* 24.0, 11.9 Hz, 1H), 2.49-2.40 (m, 2H), 2.37 (s, 3H), 2.29-2.19 (m, 1H), 2.19 - 2.00 (m, 2H). ¹³C NMR (151 MHz, DMSO-*d₆*) δ 176.69, 163.09, 159.47, 157.52, 155.72, 146.53, 139.67, 133.88, 126.46, 120.40, 118.25, 117.35, 109.27, 60.83, 36.48, 35.16, 19.11, 17.19, 14.32. HRMS (ESI) (m/z): [M+H]⁺calc. for C₂₁H₁₉F₂N₅O₂, 412.1584; found 412.1585.

### 8-(3,3-Difluorocyclopentyl)-2-((7-fluoro-1H-indazol-5-yl)amino)-5-methylpyrido[2,3-d]pyri midin-7(8H)-one (016)

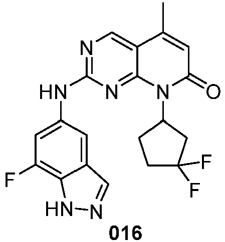

The synthesis method is described as in Example 1.

Light yellow solids, with a yield of 39%.

¹H NMR (400 MHz, DMSO-*d₆*) δ 13.57 (s, 1H), 10.15 (s, 1H), 8.85 (d, *J =* 17.4 Hz, 1H), 8.09 (s, 1H), 7.94 (s, 1H), 7.56 (d, *J =* 13.1 Hz, 1H), 6.20 (d, *J =* 33.6 Hz, 1H), 6.19-6.02 (m, 1H), 3.04 (dq, *J =* 23.9, 12.0 Hz, 1H), 2.40 (s, 3H), 2.38 - 2.18 (m, 2H), 2.18 - 1.97 (m, 3H). HRMS (ESI) (m/z): [M+H]⁺ calcd for C₂₀H₁₇F₃N₆O, 415.1496; found 415.1495.

### 2-((1H-pyrazolo[3,4-b]pyridin-5-yl)amino)-8-(3,3-difluorocyclopentyl)-5-methylpyrido[2,3-d]pyrimidin-7(8H)-one (017)

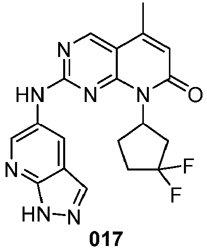

The synthesis method is described as in Example 1.

Light yellow solids, with a yield of 39%.

¹H NMR (600 MHz, DMSO-*d₆*) δ 13.61 (s, 1H), 10.18 (s, 1H), 8.87 (s, 1H), 8.70 (d, *J =* 2.2 Hz, 1H), 8.51 (s, 1H), 8.07 (s, 1H), 6.24 (s, 1H), 6.08-5.90 (m, 1H), 2.99 (tt, *J =* 44.1, 22.0 Hz, 1H), 2.49-2.39 (m, 2H), 2.40 (s, 3H), 2.32-2.17 (m, 1H), 2.15 - 1.93 (m, 2H). ¹³C NMR (151 MHz, DMSO-*d₆*) δ 163.05, 159.78, 157.70, 155.50, 154.77, 148.95, 146.57, 145.07, 143.34, 133.29, 130.17, 121.13, 117.75, 114.45, 40.50, 39.96, 36.48, 33.90, 17.24. HRMS (ESI) (m/z): [M+H]⁺ calcd for C₁₉H₁₇F₂N₇O₂,398.1542; found 398.1542.

### 2-((1H-indazol-5-yl)amino)-8-(3-hydroxycyclopentyl)-5-methylpyrido[2,3-d]pyrimidin-7(8H )-one (018)

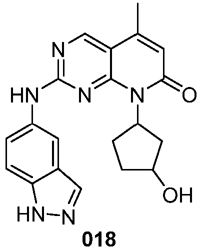

The synthesis method is described as in Example 1.

Light yellow solids, with a yield of 39%.

¹H NMR (400 MHz, DMSO-*d₆*) δ 13.00 (s, 1H), 10.04 (s, 1H), 8.85 (s, 1H), 8.20 (s, 1H), 8.02 (s, 1H), 7.56 (d, *J =* 22.2 Hz, 2H), 6.22 (s, 1H), 5.93 (s, 1H), 5.09 (d, *J =* 5.7 Hz, 1H), 4.14 (s, 1H), 2.39 (s, 3H), 2.29-2.18 (m, 1H), 2.11-2.15 (m, 2H), 1.89-1.79 (m, 3H). ¹³C NMR (151 MHz, DMSO-*d₆*) δ 163.20, 159.57, 158.24, 157.48, 155.85, 146.36, 137.18, 133.65, 133.03, 123.31, 122.01, 117.72, 110.45, 107.24, 71.75, 49.07, 37.23, 34.56, 25.56, 17.17.

### 2-((1H-indazol-5-yl)amino)-5-methyl-8-(cyclohexyl)pyrido[2,3-d]pyrimidin-7(8H)-one (019)

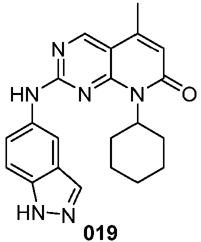

The synthesis method is described as in Example 1.

¹H NMR (400 MHz, DMSO-*d₆*) δ 13.03 (s, 1H), 10.04 (s, 1H), 8.82 (d, *J =* 22.3 Hz, 1H), 7.98 (s, 1H), 7.53 (d, *J =* 8.3 Hz, 2H), 6.19 (d, *J =* 22.3 Hz, 1H), 5.36 (d, *J =* 32.6 Hz, 1H), 2.53 (s, 2H), 2.37 (s, 3H), 1.88 (d, *J* = 30.7 Hz, 2H), 1.56 (s, 3H), 1.41 - 1.26 (m, 3H).

### 2-((1H-indazol-5-yl)amino)-5-methyl-8-(cycloheptyl)pyrido[2,3-d]pyrimidin-7(8H)-one (020)

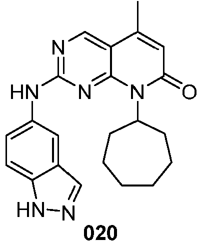

The synthesis method is described as in Example 1.

### 2-((1H-indazol-5-yl)amino)-5-methyl-8-(tetrahydrofuran-3-yl)pyrido[2,3-d]pyrimidin-7(8H)-one (021)

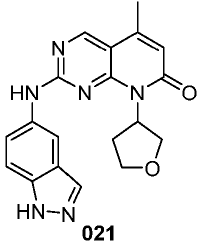

The synthesis method is described as in Example 1.

Light yellow solids, with a yield of 39%.

¹H NMR (400 MHz, DMSO-*d₆*) δ 13.00 (s, 1H), 9.99 (s, 1H), 8.83 (s, 1H), 8.13 (s, 1H), 8.01 (s, 1H), 7.64 - 7.43 (m, 2H), 6.21 (d, J = 1.0 Hz, 1H), 6.12 (s, 1H), 4.13-4.01 (m, 1H), 4.05 - 3.82 (m, 4H), 2.49 - 2.40 (m, 1H), 2.38 (s, 3H). ¹³C NMR (151 MHz, DMSO-*d₆*) δ 162.88, 159.61, 157.41, 156.04, 146.33, 137.30, 133.69, 132.88, 123.34, 122.34, 117.42, 111.07, 110.44, 107.26, 68.25, 64.34, 51.26, 28.39, 17.20.

### Example 2. Test on bioactivity

The experiment for testing the *in vitro* inhibiting effect of the compound provided in the present invention on the activity of RSK4 kinase is as follows. The method for RSK1-3 was the same as that for RSK4 (Kashem, M. A. et al. J. Biomol. Screen. 12, 70-83):
*In vitro* Enzyme Activity Assay:
All enzyme reactions were carried out at 30°C for 40 minutes. 50 µL of reaction mixture contains 40 mM Tris, pH 7.4, 10 mM MgCl₂, 0.1 mg/mL BSA, 1 mM DTT, 10 µM ATP, 0.2 µg/mL kinase, and 100 µM lipid substrate. The compound was diluted in 10% DMSO, and 5 µL of the diluted solution was added to 50 µL of the reaction, so that the final concentration of DMSO in all reactions was 1%. Kinase-Glo reagent was added to the reaction system for detection. Kinase activity was measured by quantifying the amount of ATP remaining in the solution after the kinase reaction. IC₅₀ values were calculated using nonlinear regression, and each experiment was repeated more than twice.

Test results are shown in Table 1 below.

**Table 1: Inhibiting Activity of pyridonopyrimidine compounds against RSK4**

| Number of compounds | Structure | RSK4 kinase activity IC₅₀ (nM) |
|---|---|---|
| **BI-D1870** | 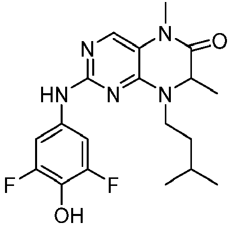 Chemical Formula: C₁₉H₂₃F₂N₅O₂ | 27±4 |
| | Exact Mass: 391.18 | |
| **001** | 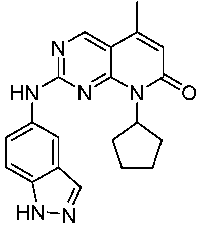 Chemical Formula: C₂₀H₂₀N₆O | 57±5 |
| | Exact Mass: 360.17 | |
| **002** | 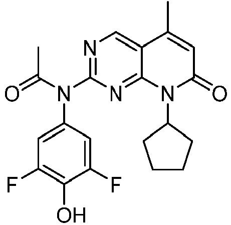 Chemical Formula: C₂₁H₂₀F₂N₄O₃ | 139±19 |
| | Exact Mass: 414.15 | |
| **003** | 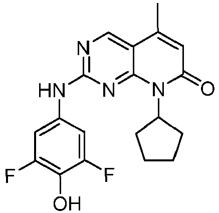 Chemical Formula: C₁₉H₁₈F₂N₄O₂ | 12±3 |
| | Exact Mass: 372.14 | |
| **004** | 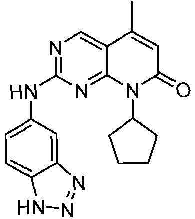 Chemical Formula: C₁₉H₁₉N₇O | 3021±673 |
| | Exact Mass: 361.17 | |
| **005** | 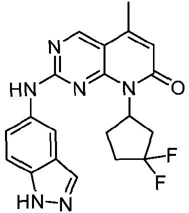 Chemical Formula: C₂₀H₁₈F₂N₆O | 14±1 |
| | Exact Mass: 396.1510 | |
| **006** | 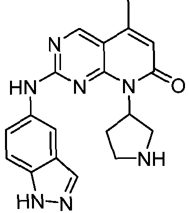 Chemical Formula: C₁₉H₁₉N₇O | 131±47 |
| | Exact Mass: 361.17 | |
| **007** | 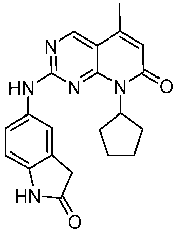 Chemical Formula: C₂₁H₂₁N₅O₂ | 17±6 |
| | Exact Mass: 375.17 | |
| **008** | 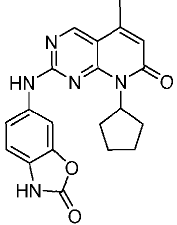 Chemical Formula: C₂₀H₁₉N₅O₃ | 10763±2027 |
| | Exact Mass: 377.15 | |
| **009** | 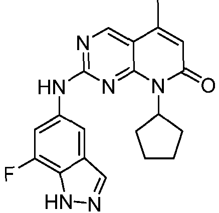 Chemical Formula: C₂₀H₁₉FN₆O | 39±8 |
| | Exact Mass: 378.16 | |
| **010** | 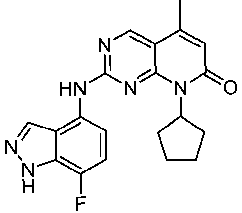 Chemical Formula: C₂₀H₁₉FN₆O | 180±16 |
| | Exact Mass: 378.16 | |
| **011** | 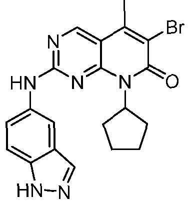 Chemical Formula: C₂₀H₁₉BrN₆O | 44±7 |
| | Exact Mass: 438.08 | |
| **012** | 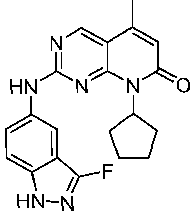 Chemical Formula: C₂₀H₁₉FN₆O | 280±54 |
| | Exact Mass: 378.16 | |
| **013** | 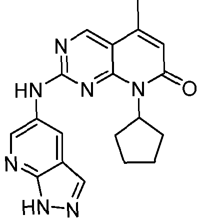 Chemical Formula: C₁₉H₁₉N₇O | 39±4 |
| | Exact Mass: 361.17 | |
| **014** | 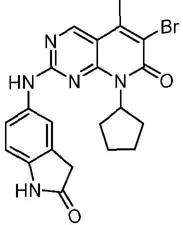 Chemical Formula: C₂₁H₂₀BrN₅O₂ | 214±20 |
| | Exact Mass: 453.08 | |
| **015** | 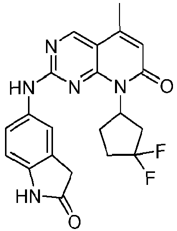 Chemical Formula: C₂₁H₁₉F₂N₅O₂ | 46±4 |
| | Exact Mass: 411.15 | |
| **016** | 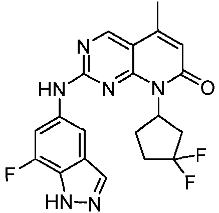 Chemical Formula: C₂₀H₁₇F₃N₆O | 13±3 |
| | Exact Mass: 414.14 | |
| **017** | 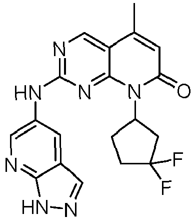 Chemical Formula: C₁₉H₁₇F₂N₇O | 12±2 |
| | Exact Mass: 397.1463 | |
| **018** | 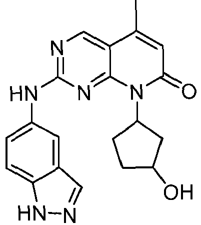 Chemical Formula: C₂₀H₂₀N₆O₂ | 30±12 |
| | Exact Mass: 376.1648 | |
| **019** | 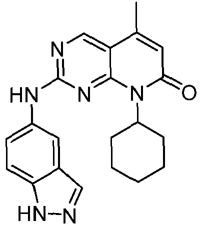 Chemical Formula: C₂₁H₂₂N₆O | 86±41 |
| | Exact Mass: 374.1855 | |
| **020** | 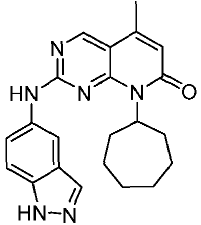 Chemical Formula: C₂₂H₂₄N₆O | 201±77 |
| | Exact Mass: 388.2012 | |
| **021** | 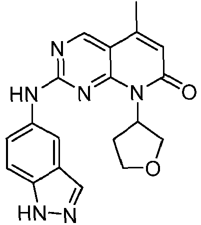 Chemical Formula: C₁₉H₁₈N₆O₂ | 392±162 |
| | Exact Mass: 362.1491 | |
| **005-1 (Peak 1 of the compound after the resolution of 005)** | 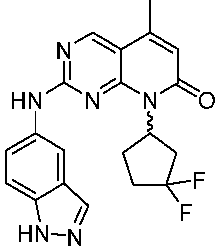 Chemical Formula: C₂₀H₁₈F₂N₆O | 11±1 |
| | Exact Mass: 396.1510 | |
| **005-2 (Peak 2 of the compound after the resolution of 005)** | 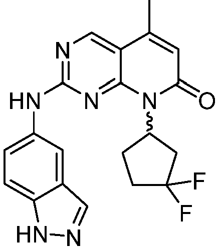 Chemical Formula: C₂₀H₁₈F₂N₆O | 6±1 |
| | Exact Mass: 396.1510 | |

### Example3. Assay on Cell biological activity

For representative compounds with excellent kinase-inhibiting activities, anti-proliferation activity tests were carried out on various esophageal squamous cell carcinoma cells such as TE10, KYSE510 and KYSE150 cells and renal cancer cells ACHN.

The specific experimental procedure is as follows: cells were digested and centrifuged at 1000 rpm for 3 minutes. The cell density was adjusted to 3×10⁴ cells/mL. 3000 cells per well was seeded, and the outermost ring was filled with culture medium. The plate was placed in a 37°C, 5% CO₂ incubator overnight to allow cell attachment. The 10 mM stock solution was diluted to 100 µM with the culture medium, and then further diluted in three-fold with the culture medium containing 1% DMSO to prepare eight concentration gradients. 10 µL of the compound was added to each well, and the remaining volume was filled with 10 µL of culture medium containing 1% DMSO. After culturing the cells for 72 hours, 10 µL of CCK8 reagent was added to each well. The cells were incubated in darkness for 2 hours, and the OD values were measured at a wavelength of 450 nM on a microplate reader. Cell viability (%) = (OD of drug-treated cells - OD of blank) / (OD of control cells - OD of blank) × 100%. After the experiment was independently repeated for three times, the variance was calculated and statistical graphs were created to analyze the effect of the drug on cell viability. IC₅₀ values were calculated by fitting with GraphPad Prism software.

Anti-cell proliferation activity assays showed that pyridonopyrimidine compounds exhibited good anti-proliferation and inhibitory activities against both renal cancer cells and esophageal squamous cell carcinoma cells, that is, activity advantages compared with BI-D1870.

**Table 2: Anti-cell proliferation activities of pyridonopyrimidine compounds**

| **Compound** | **ACHN (IC₅₀ µM)** | **TE10 (IC₅₀ µM)** | **KYSE510 (IC₅₀ µM)** | **KYSE150 (IC₅₀ µM)** |
|---|---|---|---|---|
| **003** | **3.10 ± 0.11** | **/** | **3.13 ± 1.16** | **4.08 ± 1.12** |
| **005** | **0.45 ± 0.01** | **3.28±0.88** | **2.56 ± 0.46** | **2.23 ± 0.17** |
| **007** | **1.94 ± 0.07** | **/** | **6.15 ± 0.27** | **4.65 ± 2.11** |
| **016** | **0.87 ± 0.37** | **1.70 ± 1.43** | **/** | **/** |
| **BI-D1870** | **>10** | **3.86 ± 0.76** | **11.74 ± 2.58** | **20.23 ± 1.56** |

### Example 4. Anti-cell invasion activity assay

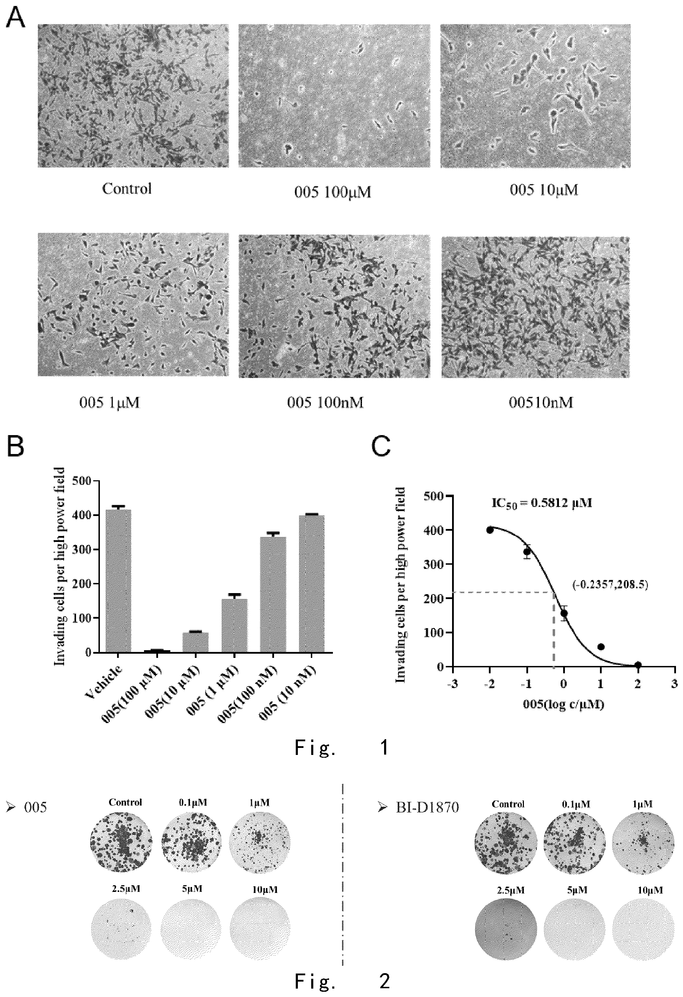

Compound 005 was subjected to Transwell experiment, and it was found that compound 005 can inhibit the invasion of esophageal squamous cell carcinoma cells TE10 in a dose-dependent manner. The results are shown in Figure 1.

### Example 5. Effects of the compound on the colony formation of esophageal squamous cell carcinoma cells

For further verifying the effect of compound 005 on the proliferation ability of TE10 cells, in this Example, a cell colony formation assay was used to further evaluate the inhibiting effect of compound 005 on the proliferation of TE10 cells. Compared with the control group, after the addition of compound 005, the number of colonies formed by TE10 cells was significantly reduced, and compound 005 could significantly inhibit the proliferation of TE10 cells at a concentration of 2.5 µM. When the concentration of compound 005 reached 5 µM and 10 µM, the proliferation of TE10 cells was almost completely inhibited, which was comparable to the ability of BI-D1870 to inhibit the colony formation of TE10 cells. The results are shown in Figure 2.

### Example 6. Influence of intracellular signaling pathways

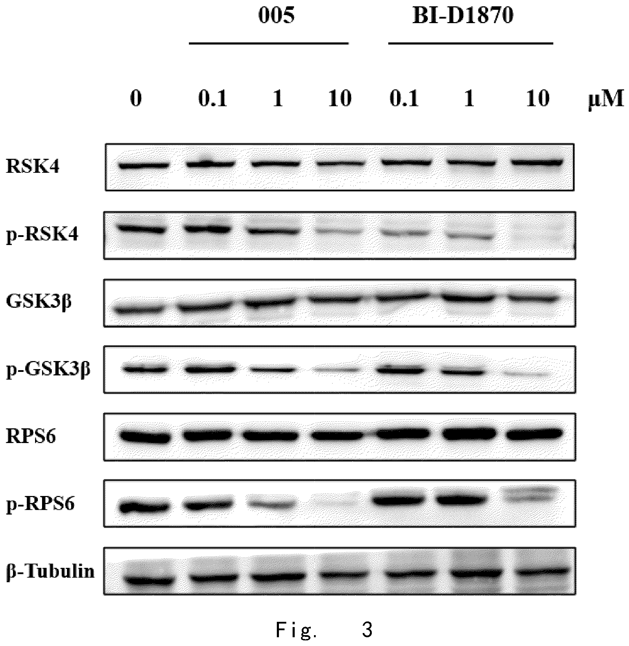

The RSK kinase family is regulated by the ERK signaling pathway and is activated under mitogenic stimulation, resulting in the phosphorylation of RPS6 at Ser235/236. When RSK4 is activated, it phosphorylates its Ser232 site, as well as the RSK family substrate RPS6 and the downstream substrate GSK3β of RSK4. As shown in Figure 3, compound 005 exhibited an influence on the RSK signaling pathway and inhibited the phosphorylation of RSK4, GSK3β, and RPS6 in a dose-dependent manner.

### Example 7. Study on the pharmacokinetic properties of compound 005

Data from the pharmacokinetic experiment showed that when compound 005 was administered via intravenous injection at a dosage of 1 mg/kg, the AUC0-t reached 530.13 ng/mL*h. When administered orally at a dosage of 10 mg/kg, the AUC0-t was as high as 3339.12 ng/mL*h. Meanwhile, the half-life of 005 was 1.97 h. It has a moderate clearance rate (3.27 L/h/kg) and *in vivo* distribution (8.42 L/kg), and the oral bioavailability reached 63%.

**Table 3 Pharmacokinetic parameters of compound 005^{a}**

| Parameter | Compound **005** | |
|---|---|---|
| | IV (1 mg/kg) | PO (10 mg/kg) |
| Tmax (h) | 0.08±0 | 0.25±0 |
| T_{1/2} (h) | 0.28±0.02 | 1.97±0.77 |
| Cmax (ng/mL) | 1357.23±105.57 | 907.95±134.07 |
| AUC₀₋ₜ (h*ng/mL) | 530.13±60.36 | 3339.12±1544.81 |
| CL_obs (L/h/kg) | 1.89±0.21 | 3.27±1.21 |
| Vss_obs (L/kg) | 0.56±0.04 | 8.42±0.58 |
| *F* (%) | -- | 63.0 |

### Discussion:

Through extensive and in-depth research, the inventors designed and synthesized a series of pyridonopyrimidine compounds that have not been reported in the literature. Molecular-level activity tests were conducted on the obtained compounds, and compounds capable of inhibiting RSK protein kinases were identified. thereby laying the foundation for treating cancers mediated by RSK.

All documents mentioned herein are cited as references in the present application as if each document is cited individually as a reference. It is further to be understood that after reading the foregoing teachings of the present invention, a skilled person can make various alterations or modifications to the present invention, and such equivalent forms will likewise fall within the scope of the claims appended to the present application.

## Claims

1. A compound as shown in Formula I, or an optical isomer, or pharmaceutically acceptable salt thereof 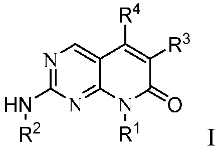
R¹ is selected from the group consisting of: a hydrogen, optionally substituted C₁-C₁₀ alkyl, optionally substituted C₃-C₈ cycloalkyl, optionally substituted C₂-C₆ alkenyl, optionally substituted C₃-C₆ cycloalkenyl, optionally substituted C₃-C₈ lactone group, optionally substituted C₁-C₁₀ amide group, optionally substituted C₅-C₁₀ aryl, optionally substituted C₃-C₈ heterocyclyl, optionally substituted C₅-C₁₀ heteroaryl;
R² is selected from the group consisting of: a hydrogen, substituted C₁-C₁₀ alkyl, C₃-C₈ cycloalkyl, optionally substituted C₅-C₁₀ aryl, optionally substituted C₃-C₈ heterocyclyl, optionally substituted C₅-C₁₀ aryl or heteroaryl fused to a five- or six- membered heterocycle;
R³ is selected from the group consisting of: a hydrogen, substituted C₁-C₁₀ alkyl, C₃-C₈ cycloalkyl, substituted C₁-C₁₀ alkyl formyl, optionally substituted C₅-C₁₀ aryl formyl, halogen, cyano, optionally substituted C₅-C₁₀ aryl, optionally substituted C₃-C₈ heterocyclyl;
R⁴ is selected from the group consisting of: a hydrogen, optionally substituted C₁-C₆ alkyl (for example, trifluoromethyl), optionally substituted C₃-C₈ cycloalkyl, optionally substituted C₁-C₁₀ alkyl formyl, optionally substituted C₅-C₁₀ aryl formyl, optionally substituted C₅-C₁₀ aryl, halogen (for example, fluorine) and optionally substituted C₃-C₈ heterocyclyl.

2. The compound of claim 1, or an optical isomer, or pharmaceutically acceptable salt thereof, wherein R¹ is selected from the group consisting of: a hydrogen, optionally substituted C₁-C₁₀ alkyl, optionally substituted C₃-C₈ cycloalkyl, optionally substituted C₅-C₁₀ aryl, and optionally substituted C₃-C₈ heterocyclyl;
R² is selected from the group consisting of: an optionally substituted C₅-C₁₀ aryl, optionally substituted C₃-C₈ heterocyclyl, and optionally substituted C₅-C₁₀ aryl or heteroaryl fused to a five- or six- membered heterocycle;
R³ is selected from the group consisting of: a hydrogen, optionally substituted C₁-C₁₀ alkyl, and optionally substituted C₃-C₈ cycloalkyl;
R⁴ is selected from the group consisting of: a hydrogen, optionally substituted C₁-C₆ alkyl (for example, trifluoromethyl), optionally substituted C₃-C₈ cycloalkyl, halogen (for example, fluorine), and optionally substituted C₃-C₈ heterocyclyl.

3. The compound of claim 1, or an optical isomer, or pharmaceutically acceptable salt thereof, wherein the compound is a compound as shown in Formula II, 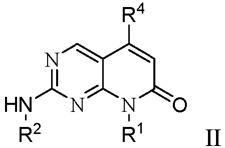
Wherein R¹ is selected from the group consisting of: a hydrogen, optionally substituted C₁-C₁₀ alkyl, optionally substituted C₃-C₈ cycloalkyl;
R² is selected from the group consisting of: an optionally substituted C₅-C₁₀ aryl, optionally substituted C₃-C₈ heterocyclyl, optionally substituted C₅-C₁₀ aryl or heteroaryl fused to a five- or six- membered heterocycle;
R⁴ is selected from the group consisting of: a hydrogen, optionally substituted C₁-C₆ alkyl (for example, trifluoromethyl), optionally substituted C₃-C₈ cycloalkyl.

4. The compound of claim 3, or an optical isomer, or pharmaceutically acceptable salt thereof, wherein R¹ is an optionally substituted C₃-C₈ cycloalkyl; preferably, a C₃-C₈ cycloalkyl substituted with one or more halogens; and more preferably, a C₃-C₈ cycloalkyl substituted with one or more F;
R² is selected from the following group: an optionally substituted C₅-C₁₀ aryl (preferably a phenyl), an optionally substituted C5-C10 aryl or heteroaryl fused to five- or six- membered heterocycle;
the C₅-C₁₀ aryl or heteroaryl fused to five- or six- membered heterocycle is: 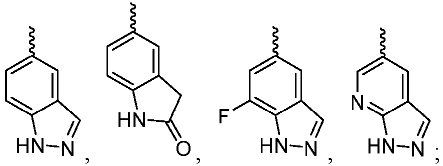
R⁴ is selected from the group consisting of: a hydrogen, optionally substituted C₁-C₆ alkyl.

5. A compound selected from the following group, or an optical isomer, or pharmaceutically acceptable salt thereof: 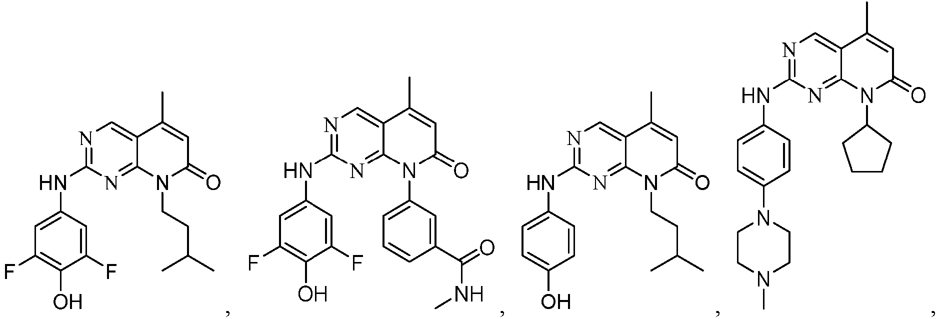 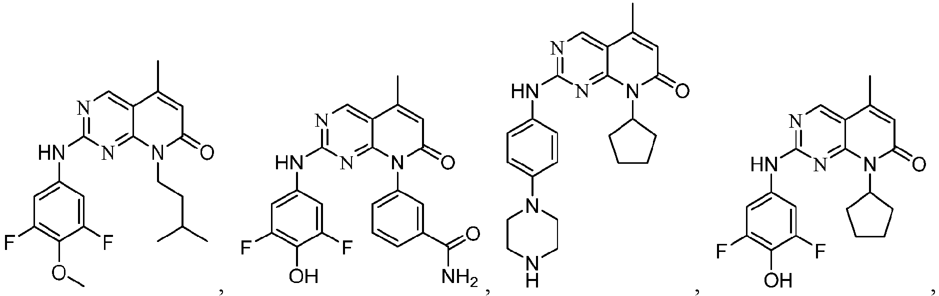 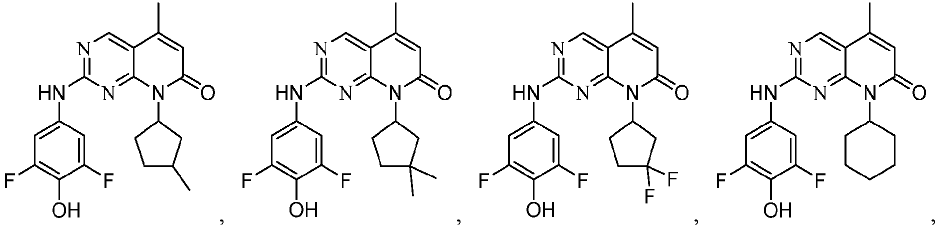 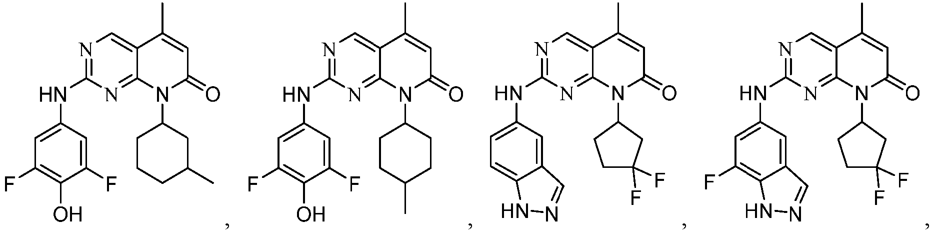 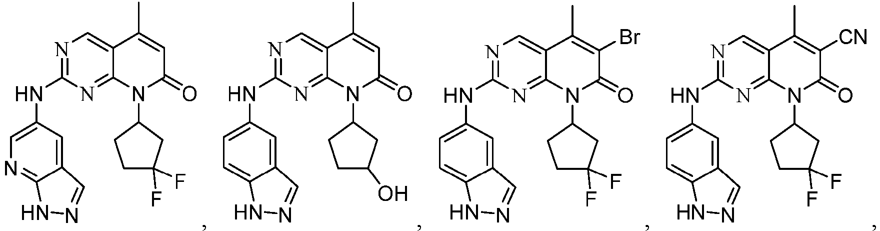 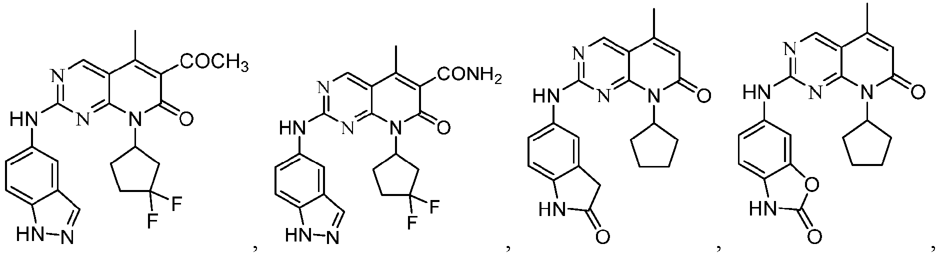 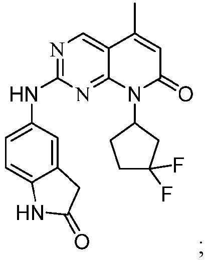 and preferably, following compounds:

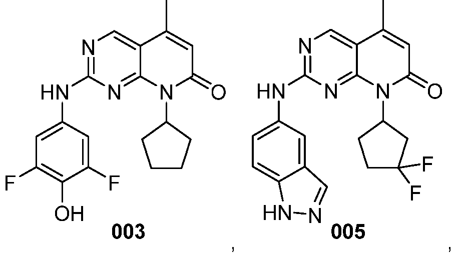

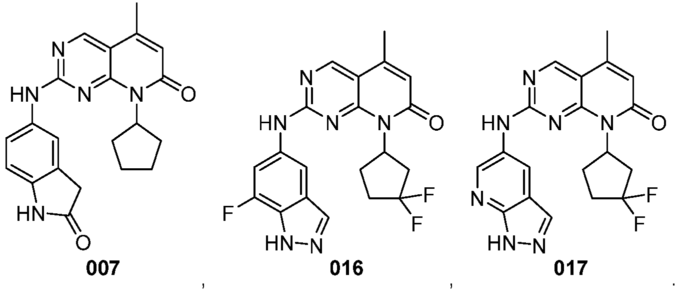

6. A pharmaceutical composition comprising the compound of any one of claims 1-5, or an optical isomer, or pharmaceutically acceptable salt thereof, and a pharmaceutically acceptable carrier or excipient is provided the present invention.

7. Use of the compound of any one of claims 1-5 in the preparation of a medicament for treating or preventing a disease mediated by RSK protein kinase, or inhibiting RSK protein kinase, or a medicament for inhibiting RSK of one of RSK1, RSK2, RSK3 and RSK4.

8. The use of claim 7, wherein the disease mediated by the RSK protein kinase is a cancer.

9. The use of claim 8, wherein the cancer is selected from the group consisting of esophageal cancer, renal cell carcinoma, pancreatic cancer, colon cancer, breast cancer, lung cancer, prostate cancer, ovarian cancer, endometrial cancer, head and neck squamous cell carcinoma, acute myeloid leukemia, and solid tumors, or breast cancer regulated by RSK1 and RSK4, ovarian cancer regulated by RSK3 and RSK4, prostate cancer regulated by RSK1 and RSK2, lung cancer regulated by RSK1, RSK2 and RSK4, head and neck squamous cell carcinoma and acute myeloid leukemia regulated by RSK2, esophageal cancer, renal cancer, endometrial cancer, colon cancer and other cancers and solid tumors regulated by RSK4.

10. A method for treating or preventing RSK-mediated diseases using the compound of any one of claims 1-5 or the pharmaceutical composition of claim 6.
